(19) Europäisches Patentamt
European Patent Office
Office européen des brevets

(11) EP 2 127 674 B1

(12) # EUROPEAN PATENT SPECIFICATION

(45) Date of publication and mention of the grant of the patent:
**24.04.2013 Bulletin 2013/17**

(21) Application number: **08720756.9**

(22) Date of filing: **19.02.2008**

(51) Int Cl.:
***A61K 38/18*** *(2006.01)* ***A61Q 7/00*** *(2006.01)*
***A61P 17/14*** *(2006.01)*

(86) International application number:
**PCT/JP2008/052781**

(87) International publication number:
**WO 2008/102783 (28.08.2008 Gazette 2008/35)**

(54) **Use of FGF18 inhibitors as hair regrowth promoter**

Verwendung von FGF-18-Hemmers als Mittel zur Förderung des Nachwachsens von Haaren

Utilisation d'inhibiteurs de FGF18 comme promoteur de la repousse des cheveux

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **19.02.2007 JP 2007038223**

(43) Date of publication of application:
**02.12.2009 Bulletin 2009/49**

(73) Proprietor: **National Institute of Advanced Industrial Science and Technology**
**Tokyo 100-8921 (JP)**

(72) Inventors:
- **IMAMURA, Toru**
  **Tsukuba-shi**
  **Ibaraki 305-8566 (JP)**
- **KIMURA, Miho**
  **Tsukuba-shi**
  **Ibaraki 305-8566 (JP)**
- **KAWANO, Mitsuko**
  **Tsukuba-shi**
  **Ibaraki 305-8566 (JP)**
- **TSUJINO, Nozomi**
  **Tsukuba-shi**
  **Ibaraki 305-8566 (JP)**
- **KURAMOCHI, Akiko**
  **Tsukuba-shi**
  **Ibaraki 305-8566 (JP)**
- **ODA, Yuko**
  **Tsukuba-shi**
  **Ibaraki 305-8566 (JP)**
- **MOTOMURA, Kaori**
  **Tsukuba-shi**
  **Ibaraki 305-8566 (JP)**
- **SUZUKI, Masashi**
  **Tsukuba-shi**
  **Ibaraki 305-8566 (JP)**
- **ASADA, Masahiro**
  **Tsukuba-shi**
  **Ibaraki 305-8566 (JP)**
- **KAMEYAMA, Azusa**
  **Tsukuba-shi**
  **Ibaraki 305-8566 (JP)**
- **TOGAYACHI, Sumie**
  **Tsukuba-shi**
  **Ibaraki 305-8566 (JP)**
- **OKA, Syuichi**
  **Tsukuba-shi**
  **Ibaraki 305-8566 (JP)**

(74) Representative: **Woods, Geoffrey Corlett et al**
**J A Kemp**
**14 South Square**
**Gray's Inn**
**London WC1R 5JJ (GB)**

(56) References cited:
**JP-A- 2003 176 213** **JP-A- 2006 117 659**

- **DATABASE WPI Week 200371 Thomson Scientific, London, GB; AN 2003-751632 XP002626940, & JP 2003 176213 A (DOKURITSU GYOSEI HOJIN SANGYO GIJUTSU SO) 24 June 2003 (2003-06-24)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 127 674 B1

- DATABASE WPI Week 200610 Thomson Scientific, London, GB; AN 2006-100942 XP002626941, & WO 2006/006270 A1 (KOBAYASHI PHARM CO LTD) 19 January 2006 (2006-01-19)
- DATABASE WPI Week 200653 Thomson Scientific, London, GB; AN 2006-521613 XP002626942, & WO 2006/030693 A1 (DOKURITSU GYOSEI HOJIN SANGYO GIJUTSU SO) 23 March 2006 (2006-03-23)
- MITSUKO KAWANO ET AL: "Comprehensive analysis of FGF and FGFR expression in skin: FGF18 is highly expressed in hair follicles and capable of inducing anagen from telogen stage hair follicles", JOURNAL OF INVESTIGATIVE DERMATOLOGY, NATURE PUBLISHING GROUP, GB, vol. 124, 1 May 2005 (2005-05-01), pages 877-885, XP003005530, ISSN: 0022-202X, DOI: DOI:10.1111/J.0022-202X.2005.23693.X
- "Hair disorders "Alopecia"" In: anonymous: "The Merk Manual of diagnosis and therapy. Eighteenth edition", 2006, Merk Research Laboratories, NJ, XP002626943, ISBN: 0911910182 pages 1005-1007, * page 1007 - column 1 * * page 1007 - column 2 *

SEQUENCE LISTING

<110> NATIONAL INSTITUTE OF ADVANCED INDUSTRIAL SCIENCE AND TECHNOLOGY

<120> HAIR REGROWTH PROMOTER

<130> N.108055

<140> EP 08720756.9
<141> 2008-02-19

<150> JP2007-38223
<151> 2007-02-19

<160> 42

<170> PatentIn version 3.5

<210> 1
<211> 549
<212> DNA
<213> Homo sapiens

<220>
<221> misc_feature
<223> Full length (-signal peptide) FGF18

<400> 1

```
atggccgagg agaacgtgga cttccgcatc cacgtggaga accagacgcg ggctcgggac      60
gatgtgagcc gtaagcagct gcggctgtac cagctctaca gccggaccag tgggaaacac     120
atccaggtcc tgggccgcag gatcagtgcc cgcggcgagg atggggacaa gtatgcccag     180
ctcctagtgg agacagacac cttcggtagt caagtccgga tcaagggcaa ggagacggaa     240
ttctacctgt gcatgaaccg caaaggcaag ctcgtgggga agcccgatgg caccagcaag     300
gagtgtgtgt tcatcgagaa ggttctggag aacaactaca cggccctgat gtcggctaag     360
tactccggct ggtacgtggg cttcaccaag aaggggcggc cgcggaaggg ccccaagacc     420
cgggagaacc agcaggacgt gcatttcatg aagcgctacc ccaaggggca gccggagctt     480
cagaagccct tcaagtacac gacggtgacc aagaggtccc gtcggatccg gcccacacac     540
cctgcctga                                                             549
```

<210> 2
<211> 182
<212> PRT
<213> Homo sapiens

<220>
<221> misc_feature
<223> Full length (-signal peptide) FGF18

<400> 2

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30
```

Tyr Ser Arg Thr Ser Gly Lys His Ile Gln Val Leu Gly Arg Arg Ile
35 40 45

Ser Ala Arg Gly Glu Asp Gly Asp Lys Tyr Ala Gln Leu Leu Val Glu
50 55 60

Thr Asp Thr Phe Gly Ser Gln Val Arg Ile Lys Gly Lys Glu Thr Glu
65 70 75 80

Phe Tyr Leu Cys Met Asn Arg Lys Gly Lys Leu Val Gly Lys Pro Asp
85 90 95

Gly Thr Ser Lys Glu Cys Val Phe Ile Glu Lys Val Leu Glu Asn Asn
100 105 110

Tyr Thr Ala Leu Met Ser Ala Lys Tyr Ser Gly Trp Tyr Val Gly Phe
115 120 125

Thr Lys Lys Gly Arg Pro Arg Lys Gly Pro Lys Thr Arg Glu Asn Gln
130 135 140

Gln Asp Val His Phe Met Lys Arg Tyr Pro Lys Gly Gln Pro Glu Leu
145 150 155 160

Gln Lys Pro Phe Lys Tyr Thr Thr Val Thr Lys Arg Ser Arg Arg Ile
165 170 175

Arg Pro Thr His Pro Ala
180

<210> 3
<211> 549
<212> DNA
<213> Mus musculus

<220>
<221> misc_feature
<223> Full length (-signal peptide) FGF18

<400> 3

atggccgagg agaatgtgga cttccgcatc cacgtggaga accagacgcg ggctcgagat 60
gatgtgagtc ggaagcagct gcgcttgtac cagctctata gcaggaccag tgggaagcac 120
attcaagtcc tgggccgtag gatcagtgcc cgtggcgagg acggggacaa gtatgcccag 180
ctcctagtgg agacagatac cttcgggagt caagtccgga tcaagggcaa ggagacagaa 240
ttctacctgt gtatgaaccg aaaaggcaag ctcgtgggga agcctgatgg tactagcaag 300
gagtgcgtgt tcattgagaa ggttctggaa aacaactaca cggccctgat gtctgccaag 360
tactctggtt ggtatgtggg cttcaccaag aaggggcggc ctcgcaaggg tcccaagacc 420
cgcgagaacc agcaagatgt acacttcatg aagcgttacc ccaagggaca ggccgagctg 480

cagaagccct tcaaatacac cacagtcacc aagcgatccc ggcggatccg ccccactcac 540
cccggctag 549

<210> 4
<211> 537
<212> DNA
<213> Artificial Sequence

<220>
<223> truncation mutant FGF18(d4)

<400> 4

atggtggact tccgcatcca cgtggagaac cagacgcggg ctcgagatga tgtgagtcgg 60
aagcagctgc gcttgtacca gctctatagc aggaccagtg ggaagcacat tcaagtcctg 120
ggccgtagga tcagtgcccg tggcgaggac ggggacaagt atgcccagct cctagtggag 180
acagatacct tcgggagtca agtccggatc aagggcaagg agacagaatt ctacctgtgt 240
atgaaccgaa aaggcaagct cgtggggaag cctgatggta ctagcaagga gtgcgtgttc 300
attgagaagg ttctggaaaa caactacacg gccctgatgt ctgccaagta ctctggttgg 360
tatgtgggct tcaccaagaa ggggcggcct cgcaagggtc ccaagacccg cgagaaccag 420
caagatgtac acttcatgaa gcgttacccc aagggacagg ccgagctgca gaagcccttc 480
aaatacacca cagtcaccaa gcgatcccgg cggatccgcc ccactcaccc cggctag 537

<210> 5
<211> 513
<212> DNA
<213> Artificial Sequence

<220>
<223> truncation mutant FGF18(d12)

<400> 5

```
atgaaccaga cgcgggctcg agatgatgtg agtcggaagc agctgcgctt gtaccagctc     60
tatagcagga ccagtgggaa gcacattcaa gtcctgggcc gtaggatcag tgcccgtggc    120
gaggacgggg acaagtatgc ccagctccta gtggagacag ataccttcgg gagtcaagtc    180
cggatcaagg gcaaggagac agaattctac ctgtgtatga accgaaaagg caagctcgtg    240
gggaagcctg atggtactag caaggagtgc gtgttcattg agaaggttct ggaaaacaac    300
tacacggccc tgatgtctgc caagtactct ggttggtatg tgggcttcac caagaagggg    360
cggcctcgca agggtcccaa gacccgcgag aaccagcaag atgtacactt catgaagcgt    420
taccccaagg gacaggccga gctgcagaag cccttcaaat acaccacagt caccaagcga    480
tcccggcgga tccgccccac tcaccccggc tag                                 513
```

<210> 6
<211> 501
<212> DNA
<213> Artificial Sequence

<220>
<223> truncation mutant FGF18(d16)

<400> 6

```
atggctcgag atgatgtgag tcggaagcag ctgcgcttgt accagctcta tagcaggacc     60
agtgggaagc acattcaagt cctgggccgt aggatcagtg cccgtggcga ggacggggac    120
aagtatgccc agctcctagt ggagacagat accttcggga gtcaagtccg gatcaagggc    180
aaggagacag aattctacct gtgtatgaac cgaaaaggca agctcgtggg gaagcctgat    240
ggtactagca aggagtgcgt gttcattgag aaggttctgg aaaacaacta cacggccctg    300
atgtctgcca agtactctgg ttggtatgtg ggcttcacca agaaggggcg gcctcgcaag    360
ggtcccaaga cccgcgagaa ccagcaagat gtacacttca tgaagcgtta ccccaaggga    420
caggccgagc tgcagaagcc cttcaaatac accacagtca ccaagcgatc ccggcggatc    480
cgccccactc accccggcta g                                              501
```

<210> 7
<211> 495
<212> DNA
<213> Artificial Sequence

<220>
<223> truncation mutant FGF18(d18)

<400> 7

```
atggatgatg tgagtcggaa gcagctgcgc ttgtaccagc tctatagcag gaccagtggg      60
aagcacattc aagtcctggg ccgtaggatc agtgcccgtg gcgaggacgg ggacaagtat     120
gcccagctcc tagtggagac agataccttc gggagtcaag tccggatcaa gggcaaggag     180
acagaattct acctgtgtat gaaccgaaaa ggcaagctcg tggggaagcc tgatggtact     240
agcaaggagt gcgtgttcat tgagaaggtt ctggaaaaca actacacggc cctgatgtct     300
gccaagtact ctggttggta tgtgggcttc accaagaagg ggcggcctcg caagggtccc     360
aagacccgcg agaaccagca agatgtacac ttcatgaagc gttaccccaa gggacaggcc     420
gagctgcaga agcccttcaa atacaccaca gtcaccaagc gatcccggcg gatccgcccc     480
actcaccccg gctag                                                     495
```

<210> 8
<211> 483
<212> DNA
<213> Artificial Sequence

<220>
<223> truncation mutant FGF18(d22)

<400> 8

```
atgcggaagc agctgcgctt gtaccagctc tatagcagga ccagtgggaa gcacattcaa      60
gtcctgggcc gtaggatcag tgcccgtggc gaggacgggg acaagtatgc ccagctccta     120
gtggagacag ataccttcgg gagtcaagtc cggatcaagg gcaaggagac agaattctac     180
ctgtgtatga accgaaaagg caagctcgtg gggaagcctg atggtactag caaggagtgc     240
gtgttcattg agaaggttct ggaaaacaac tacacggccc tgatgtctgc caagtactct     300
ggttggtatg tgggcttcac caagaagggg cggcctcgca agggtcccaa gacccgcgag     360
aaccagcaag atgtacactt catgaagcgt taccccaagg gacaggccga gctgcagaag     420
cccttcaaat acaccacagt caccaagcga tcccggcgga tccgccccac tcaccccggc     480
tag                                                                  483
```

<210> 9
<211> 438
<212> DNA
<213> Artificial Sequence

<220>
<223> truncation mutant FGF18(d37)

<400> 9

```
atgaagcaca ttcaagtcct gggccgtagg atcagtgccc gtggcgagga cggggacaag      60
tatgcccagc tcctagtgga gacagatacc ttcgggagtc aagtccggat caagggcaag     120
gagacagaat tctacctgtg tatgaaccga aaaggcaagc tcgtggggaa gcctgatggt     180
actagcaagg agtgcgtgtt cattgagaag gttctggaaa acaactacac ggccctgatg     240
tctgccaagt actctggttg gtatgtgggc ttcaccaaga aggggcggcc tcgcaagggt     300
cccaagaccc gcgagaacca gcaagatgta cacttcatga agcgttaccc caagggacag     360
gccgagctgc agaagccctt caaatacacc acagtcacca agcgatcccg gcggatccgc     420
cccactcacc ccggctag                                                   438
```

<210> 10
<211> 405
<212> DNA
<213> Artificial Sequence

<220>
<223> truncation mutant FGF18(d48)

<400> 10

```
atggcccgtg gcgaggacgg ggacaagtat gcccagctcc tagtggagac agataccttc      60
gggagtcaag tccggatcaa gggcaaggag acagaattct acctgtgtat gaaccgaaaa     120
ggcaagctcg tggggaagcc tgatggtact agcaaggagt gcgtgttcat tgagaaggtt     180
ctggaaaaca actacacggc cctgatgtct gccaagtact ctggttggta tgtgggcttc     240
accaagaagg ggcggcctcg caagggtccc aagacccgcg agaaccagca agatgtacac     300
ttcatgaagc gttaccccaa gggacaggcc gagctgcaga agcccttcaa atacaccaca     360
gtcaccaagc gatcccggcg gatccgcccc actcaccccg gctag                     405
```

<210> 11
<211> 348
<212> DNA
<213> Artificial Sequence

<220>
<223> truncation mutant FGF18(d67)

<400> 11

```
atggggagtc aagtccggat caagggcaag gagacagaat tctacctgtg tatgaaccga      60
aaaggcaagc tcgtggggaa gcctgatggt actagcaagg agtgcgtgtt cattgagaag     120
gttctggaaa acaactacac ggccctgatg tctgccaagt actctggttg gtatgtgggc     180
ttcaccaaga aggggcggcc tcgcaagggt cccaagaccc gcgagaacca gcaagatgta     240
cacttcatga agcgttaccc caagggacag gccgagctgc agaagccctt caaatacacc     300
acagtcacca agcgatcccg gcggatccgc cccactcacc ccggctag                  348
```

<210> 12
<211> 318
<212> DNA
<213> Artificial Sequence

<220>
<223> truncation mutant FGF18(d77)

<400> 12

```
atgacagaat tctacctgtg tatgaaccga aaaggcaagc tcgtggggaa gcctgatggt      60
actagcaagg agtgcgtgtt cattgagaag gttctggaaa acaactacac ggccctgatg     120
tctgccaagt actctggttg gtatgtgggc ttcaccaaga aggggcggcc tcgcaagggt     180
cccaagaccc gcgagaacca gcaagatgta cacttcatga agcgttaccc caagggacag     240
gccgagctgc agaagccctt caaatacacc acagtcacca agcgatcccg gcggatccgc     300
cccactcacc ccggctag                                                   318
```

<210> 13
<211> 264
<212> DNA
<213> Artificial Sequence

<220>
<223> truncation mutant FGF18(d95)

<400> 13

```
atgggtacta gcaaggagtg cgtgttcatt gagaaggttc tggaaaacaa ctacacggcc      60
ctgatgtctg ccaagtactc tggttggtat gtgggcttca ccaagaaggg gcggcctcgc     120
aagggtccca agacccgcga gaaccagcaa gatgtacact tcatgaagcg ttaccccaag     180
ggacaggccg agctgcagaa gcccttcaaa tacaccacag tcaccaagcg atcccggcgg     240
atccgcccca ctcaccccgg ctag                                            264
```

<210> 14
<211> 182
<212> PRT
<213> Mus musculus

<220>
<221> misc_feature
<223> Full length (-signal peptide) FGF18

<400> 14

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys Tyr Ala Gln Leu Leu Val Glu
    50                  55                  60

Thr Asp Thr Phe Gly Ser Gln Val Arg Ile Lys Gly Lys Glu Thr Glu
65                  70                  75                  80

Phe Tyr Leu Cys Met Asn Arg Lys Gly Lys Leu Val Gly Lys Pro Asp
                85                  90                  95

Gly Thr Ser Lys Glu Cys Val Phe Ile Glu Lys Val Leu Glu Asn Asn
            100                 105                 110

Tyr Thr Ala Leu Met Ser Ala Lys Tyr Ser Gly Trp Tyr Val Gly Phe
        115                 120                 125

Thr Lys Lys Gly Arg Pro Arg Lys Gly Pro Lys Thr Arg Glu Asn Gln
    130                 135                 140

Gln Asp Val His Phe Met Lys Arg Tyr Pro Lys Gly Gln Ala Glu Leu
145                 150                 155                 160

Gln Lys Pro Phe Lys Tyr Thr Thr Val Thr Lys Arg Ser Arg Arg Ile
                165                 170                 175

Arg Pro Thr His Pro Gly
            180
```

<210> 15
<211> 178
<212> PRT
<213> Artificial Sequence

<220>
<223> truncation mutant FGF18(d4)

<400> 15

```
Met Val Asp Phe Arg Ile His Val Glu Asn Gln Thr Arg Ala Arg Asp
1               5                   10                  15

Asp Val Ser Arg Lys Gln Leu Arg Leu Tyr Gln Leu Tyr Ser Arg Thr
            20                  25                  30

Ser Gly Lys His Ile Gln Val Leu Gly Arg Arg Ile Ser Ala Arg Gly
        35                  40                  45

Glu Asp Gly Asp Lys Tyr Ala Gln Leu Leu Val Glu Thr Asp Thr Phe
    50                  55                  60

Gly Ser Gln Val Arg Ile Lys Gly Lys Glu Thr Glu Phe Tyr Leu Cys
65                  70                  75                  80

Met Asn Arg Lys Gly Lys Leu Val Gly Lys Pro Asp Gly Thr Ser Lys
                85                  90                  95

Glu Cys Val Phe Ile Glu Lys Val Leu Glu Asn Asn Tyr Thr Ala Leu
            100                 105                 110

Met Ser Ala Lys Tyr Ser Gly Trp Tyr Val Gly Phe Thr Lys Lys Gly
        115                 120                 125

Arg Pro Arg Lys Gly Pro Lys Thr Arg Glu Asn Gln Gln Asp Val His
    130                 135                 140

Phe Met Lys Arg Tyr Pro Lys Gly Gln Ala Glu Leu Gln Lys Pro Phe
145                 150                 155                 160

Lys Tyr Thr Thr Val Thr Lys Arg Ser Arg Arg Ile Arg Pro Thr His
                165                 170                 175

Pro Gly
```

<210> 16
<211> 170
<212> PRT
<213> Artificial Sequence

<220>
<223> truncation mutant FGF18 (d12)

<400> 16

```
Met Asn Gln Thr Arg Ala Arg Asp Asp Val Ser Arg Lys Gln Leu Arg
1               5                   10                  15

Leu Tyr Gln Leu Tyr Ser Arg Thr Ser Gly Lys His Ile Gln Val Leu
            20                  25                  30

Gly Arg Arg Ile Ser Ala Arg Gly Glu Asp Gly Asp Lys Tyr Ala Gln
        35                  40                  45

Leu Leu Val Glu Thr Asp Thr Phe Gly Ser Gln Val Arg Ile Lys Gly
    50                  55                  60

Lys Glu Thr Glu Phe Tyr Leu Cys Met Asn Arg Lys Gly Lys Leu Val
65                  70                  75                  80

Gly Lys Pro Asp Gly Thr Ser Lys Glu Cys Val Phe Ile Glu Lys Val
                85                  90                  95

Leu Glu Asn Asn Tyr Thr Ala Leu Met Ser Ala Lys Tyr Ser Gly Trp
            100                 105                 110

Tyr Val Gly Phe Thr Lys Lys Gly Arg Pro Arg Lys Gly Pro Lys Thr
        115                 120                 125

Arg Glu Asn Gln Gln Asp Val His Phe Met Lys Arg Tyr Pro Lys Gly
    130                 135                 140

Gln Ala Glu Leu Gln Lys Pro Phe Lys Tyr Thr Thr Val Thr Lys Arg
145                 150                 155                 160

Ser Arg Arg Ile Arg Pro Thr His Pro Gly
                165                 170
```

<210> 17
<211> 166
<212> PRT
<213> Artificial Sequence

<220>
<223> truncation mutant FGF18(d16)

<400> 17

```
Met Ala Arg Asp Asp Val Ser Arg Lys Gln Leu Arg Leu Tyr Gln Leu
1               5                   10                  15

Tyr Ser Arg Thr Ser Gly Lys His Ile Gln Val Leu Gly Arg Arg Ile
            20                  25                  30

Ser Ala Arg Gly Glu Asp Gly Asp Lys Tyr Ala Gln Leu Leu Val Glu
        35                  40                  45

Thr Asp Thr Phe Gly Ser Gln Val Arg Ile Lys Gly Lys Glu Thr Glu
    50                  55                  60

Phe Tyr Leu Cys Met Asn Arg Lys Gly Lys Leu Val Gly Lys Pro Asp
65                  70                  75                  80

Gly Thr Ser Lys Glu Cys Val Phe Ile Glu Lys Val Leu Glu Asn Asn
                85                  90                  95

Tyr Thr Ala Leu Met Ser Ala Lys Tyr Ser Gly Trp Tyr Val Gly Phe
            100                 105                 110

Thr Lys Lys Gly Arg Pro Arg Lys Gly Pro Lys Thr Arg Glu Asn Gln
        115                 120                 125

Gln Asp Val His Phe Met Lys Arg Tyr Pro Lys Gly Gln Ala Glu Leu
    130                 135                 140

Gln Lys Pro Phe Lys Tyr Thr Thr Val Thr Lys Arg Ser Arg Arg Ile
145                 150                 155                 160

Arg Pro Thr His Pro Gly
                165
```

<210> 18
<211> 164
<212> PRT
<213> Artificial Sequence

<220>
<223> truncation mutant FGF18(d18)

<400> 18

Met Asp Asp Val Ser Arg Lys Gln Leu Arg Leu Tyr Gln Leu Tyr Ser
1 5 10 15

Arg Thr Ser Gly Lys His Ile Gln Val Leu Gly Arg Arg Ile Ser Ala
20 25 30

Arg Gly Glu Asp Gly Asp Lys Tyr Ala Gln Leu Leu Val Glu Thr Asp
35 40 45

Thr Phe Gly Ser Gln Val Arg Ile Lys Gly Lys Glu Thr Glu Phe Tyr
50 55 60

Leu Cys Met Asn Arg Lys Gly Lys Leu Val Gly Lys Pro Asp Gly Thr
65 70 75 80

Ser Lys Glu Cys Val Phe Ile Glu Lys Val Leu Glu Asn Asn Tyr Thr
85 90 95

Ala Leu Met Ser Ala Lys Tyr Ser Gly Trp Tyr Val Gly Phe Thr Lys
100 105 110

Lys Gly Arg Pro Arg Lys Gly Pro Lys Thr Arg Glu Asn Gln Gln Asp
115 120 125

Val His Phe Met Lys Arg Tyr Pro Lys Gly Gln Ala Glu Leu Gln Lys
130 135 140

Pro Phe Lys Tyr Thr Thr Val Thr Lys Arg Ser Arg Arg Ile Arg Pro
145 150 155 160

Thr His Pro Gly

<210> 19
<211> 160
<212> PRT
<213> Artificial Sequence

<220>
<223> truncation mutant FGF18(d22)

<400> 19

Met Arg Lys Gln Leu Arg Leu Tyr Gln Leu Tyr Ser Arg Thr Ser Gly
1 5 10 15

Lys His Ile Gln Val Leu Gly Arg Arg Ile Ser Ala Arg Gly Glu Asp
20 25 30

Gly Asp Lys Tyr Ala Gln Leu Leu Val Glu Thr Asp Thr Phe Gly Ser
35 40 45

Gln Val Arg Ile Lys Gly Lys Glu Thr Glu Phe Tyr Leu Cys Met Asn
50 55 60

Arg Lys Gly Lys Leu Val Gly Lys Pro Asp Gly Thr Ser Lys Glu Cys
65 70 75 80

Val Phe Ile Glu Lys Val Leu Glu Asn Asn Tyr Thr Ala Leu Met Ser
85 90 95

Ala Lys Tyr Ser Gly Trp Tyr Val Gly Phe Thr Lys Lys Gly Arg Pro
100 105 110

Arg Lys Gly Pro Lys Thr Arg Glu Asn Gln Gln Asp Val His Phe Met
115 120 125

Lys Arg Tyr Pro Lys Gly Gln Ala Glu Leu Gln Lys Pro Phe Lys Tyr
130 135 140

Thr Thr Val Thr Lys Arg Ser Arg Arg Ile Arg Pro Thr His Pro Gly
145 150 155 160

<210> 20
<211> 145
<212> PRT
<213> Artificial Sequence

<220>
<223> truncation mutant FGF18(d37)

<400> 20

Met Lys His Ile Gln Val Leu Gly Arg Arg Ile Ser Ala Arg Gly Glu
1 5 10 15

Asp Gly Asp Lys Tyr Ala Gln Leu Leu Val Glu Thr Asp Thr Phe Gly
20 25 30

Ser Gln Val Arg Ile Lys Gly Lys Glu Thr Glu Phe Tyr Leu Cys Met
35 40 45

```
Asn Arg Lys Gly Lys Leu Val Gly Lys Pro Asp Gly Thr Ser Lys Glu
    50                  55                  60

Cys Val Phe Ile Glu Lys Val Leu Glu Asn Asn Tyr Thr Ala Leu Met
65                  70                  75                  80

Ser Ala Lys Tyr Ser Gly Trp Tyr Val Gly Phe Thr Lys Lys Gly Arg
                85                  90                  95

Pro Arg Lys Gly Pro Lys Thr Arg Glu Asn Gln Gln Asp Val His Phe
            100                 105                 110

Met Lys Arg Tyr Pro Lys Gly Gln Ala Glu Leu Gln Lys Pro Phe Lys
        115                 120                 125

Tyr Thr Thr Val Thr Lys Arg Ser Arg Arg Ile Arg Pro Thr His Pro
    130                 135                 140

Gly
145
```

<210> 21
<211> 134
<212> PRT
<213> Artificial Sequence

<220>
<223> truncation mutant FGF18(d48)

<400> 21

```
Met Ala Arg Gly Glu Asp Gly Asp Lys Tyr Ala Gln Leu Leu Val Glu
1               5                   10                  15

Thr Asp Thr Phe Gly Ser Gln Val Arg Ile Lys Gly Lys Glu Thr Glu
            20                  25                  30

Phe Tyr Leu Cys Met Asn Arg Lys Gly Lys Leu Val Gly Lys Pro Asp
        35                  40                  45

Gly Thr Ser Lys Glu Cys Val Phe Ile Glu Lys Val Leu Glu Asn Asn
    50                  55                  60

Tyr Thr Ala Leu Met Ser Ala Lys Tyr Ser Gly Trp Tyr Val Gly Phe
65                  70                  75                  80

Thr Lys Lys Gly Arg Pro Arg Lys Gly Pro Lys Thr Arg Glu Asn Gln
                85                  90                  95

Gln Asp Val His Phe Met Lys Arg Tyr Pro Lys Gly Gln Ala Glu Leu
            100                 105                 110

Gln Lys Pro Phe Lys Tyr Thr Thr Val Thr Lys Arg Ser Arg Arg Ile
        115                 120                 125

                    Arg Pro Thr His Pro Gly
                        130
```

<210> 22
<211> 115
<212> PRT
<213> Artificial Sequence

<220>
<223> truncation mutant FGF18(d67)

<400> 22

```
Met Gly Ser Gln Val Arg Ile Lys Gly Lys Glu Thr Glu Phe Tyr Leu
1               5                   10                  15

Cys Met Asn Arg Lys Gly Lys Leu Val Gly Lys Pro Asp Gly Thr Ser
            20                  25                  30

Lys Glu Cys Val Phe Ile Glu Lys Val Leu Glu Asn Asn Tyr Thr Ala
        35                  40                  45

Leu Met Ser Ala Lys Tyr Ser Gly Trp Tyr Val Gly Phe Thr Lys Lys
    50                  55                  60

Gly Arg Pro Arg Lys Gly Pro Lys Thr Arg Glu Asn Gln Gln Asp Val
65                  70                  75                  80

His Phe Met Lys Arg Tyr Pro Lys Gly Gln Ala Glu Leu Gln Lys Pro
                85                  90                  95

Phe Lys Tyr Thr Thr Val Thr Lys Arg Ser Arg Arg Ile Arg Pro Thr
            100                 105                 110

His Pro Gly
        115
```

<210> 23
<211> 105
<212> PRT
<213> Artificial Sequence

<220>
<223> truncation mutant FGF18(d77)

<400> 23

```
Met Thr Glu Phe Tyr Leu Cys Met Asn Arg Lys Gly Lys Leu Val Gly
1               5                   10                  15

Lys Pro Asp Gly Thr Ser Lys Glu Cys Val Phe Ile Glu Lys Val Leu
            20                  25                  30

Glu Asn Asn Tyr Thr Ala Leu Met Ser Ala Lys Tyr Ser Gly Trp Tyr
        35                  40                  45
```

Val Gly Phe Thr Lys Lys Gly Arg Pro Arg Lys Gly Pro Lys Thr Arg
    50                  55                  60

Glu Asn Gln Gln Asp Val His Phe Met Lys Arg Tyr Pro Lys Gly Gln
65                  70                  75                  80

Ala Glu Leu Gln Lys Pro Phe Lys Tyr Thr Thr Val Thr Lys Arg Ser
                85                  90                  95

Arg Arg Ile Arg Pro Thr His Pro Gly
            100                 105

<210> 24
<211> 87
<212> PRT
<213> Artificial Sequence

<220>
<223> truncation mutant FGF18(d95)

<400> 24

Met Gly Thr Ser Lys Glu Cys Val Phe Ile Glu Lys Val Leu Glu Asn
1               5                   10                  15

Asn Tyr Thr Ala Leu Met Ser Ala Lys Tyr Ser Gly Trp Tyr Val Gly
            20                  25                  30

Phe Thr Lys Lys Gly Arg Pro Arg Lys Gly Pro Lys Thr Arg Glu Asn
        35                  40                  45

Gln Gln Asp Val His Phe Met Lys Arg Tyr Pro Lys Gly Gln Ala Glu
    50                  55                  60

Leu Gln Lys Pro Phe Lys Tyr Thr Thr Val Thr Lys Arg Ser Arg Arg
65                  70                  75                  80

Ile Arg Pro Thr His Pro Gly
                85

<210> 25
<211> 474
<212> DNA
<213> Artificial Sequence

<220>
<223> truncation mutant FGF18(dc25)

<400> 25

atggccgagg agaatgtgga cttccgcatc cacgtggaga accagacgcg ggctcgagat 60

gatgtgagtc ggaagcagct gcgcttgtac cagctctata gcaggaccag tgggaagcac 120

attcaagtcc tgggccgtag gatcagtgcc cgtggcgagg acggggacaa gtatgcccag 180

ctcctagtgg agacagatac cttcgggagt caagtccgga tcaagggcaa ggagacagaa 240

ttctacctgt gtatgaaccg aaaaggcaag ctcgtgggga agcctgatgg tactagcaag 300

gagtgcgtgt tcattgagaa ggttctggaa aacaactaca cggccctgat gtctgccaag 360

tactctggtt ggtatgtggg cttcaccaag aaggggcggc ctcgcaaggg tcccaagacc 420

cgcgagaacc agcaagatgt acacttcatg aagcgttacc ccaagggaca gtag 474

<210> 26
<211> 420
<212> DNA
<213> Artificial Sequence

<220>
<223> truncation mutant FGF18(dc43)

<400> 26

atggccgagg agaatgtgga cttccgcatc cacgtggaga accagacgcg ggctcgagat 60

gatgtgagtc ggaagcagct gcgcttgtac cagctctata gcaggaccag tgggaagcac 120

attcaagtcc tgggccgtag gatcagtgcc cgtggcgagg acggggacaa gtatgcccag 180

ctcctagtgg agacagatac cttcgggagt caagtccgga tcaagggcaa ggagacagaa 240

ttctacctgt gtatgaaccg aaaaggcaag ctcgtgggga agcctgatgg tactagcaag 300

gagtgcgtgt tcattgagaa ggttctggaa aacaactaca cggccctgat gtctgccaag 360

tactctggtt ggtatgtggg cttcaccaag aaggggcggc ctcgcaaggg tcccaagtag 420

<210> 27
<211> 378
<212> DNA
<213> Artificial Sequence

<220>
<223> truncation mutant FGF18(dc57)

<400> 27

atggccgagg agaatgtgga cttccgcatc cacgtggaga accagacgcg ggctcgagat 60

gatgtgagtc ggaagcagct gcgcttgtac cagctctata gcaggaccag tgggaagcac 120

attcaagtcc tgggccgtag gatcagtgcc cgtggcgagg acggggacaa gtatgcccag 180

ctcctagtgg agacagatac cttcgggagt caagtccgga tcaagggcaa ggagacagaa 240

ttctacctgt gtatgaaccg aaaaggcaag ctcgtgggga agcctgatgg tactagcaag 300

gagtgcgtgt tcattgagaa ggttctggaa aacaactaca cggccctgat gtctgccaag 360

tactctggtt ggtattag 378

<210> 28
<211> 348
<212> DNA
<213> Artificial Sequence

<220>
<223> truncation mutant FGF18(dc67)

<400> 28

atggccgagg agaatgtgga cttccgcatc cacgtggaga accagacgcg ggctcgagat 60

gatgtgagtc ggaagcagct gcgcttgtac cagctctata gcaggaccag tgggaagcac 120

attcaagtcc tgggccgtag gatcagtgcc cgtggcgagg acggggacaa gtatgcccag 180

ctcctagtgg agacagatac cttcgggagt caagtccgga tcaagggcaa ggagacagaa 240

ttctacctgt gtatgaaccg aaaaggcaag ctcgtgggga agcctgatgg tactagcaag 300

gagtgcgtgt tcattgagaa ggttctggaa aacaactaca cggcctag 348

<210> 29
<211> 303
<212> DNA
<213> Artificial Sequence

<220>
<223> truncation mutant FGF18(dc82)

<400> 29

atggccgagg agaatgtgga cttccgcatc cacgtggaga accagacgcg ggctcgagat 60

gatgtgagtc ggaagcagct gcgcttgtac cagctctata gcaggaccag tgggaagcac 120

attcaagtcc tgggccgtag gatcagtgcc cgtggcgagg acggggacaa gtatgcccag 180

ctcctagtgg agacagatac cttcgggagt caagtccgga tcaagggcaa ggagacagaa 240

ttctacctgt gtatgaaccg aaaaggcaag ctcgtgggga agcctgatgg tactagcaag 300

tag 303

<210> 30

<211> 267
<212> DNA
<213> Artificial Sequence

<220>
<223> truncation mutant FGF18(dc94)

<400> 30

```
atggccgagg agaatgtgga cttccgcatc cacgtggaga accagacgcg ggctcgagat      60
gatgtgagtc ggaagcagct gcgcttgtac cagctctata gcaggaccag tgggaagcac     120
attcaagtcc tgggccgtag gatcagtgcc cgtggcgagg acggggacaa gtatgcccag     180
ctcctagtgg agacagatac cttcgggagt caagtccgga tcaagggcaa ggagacagaa     240
ttctacctgt gtatgaaccg aaaatga                                         267
```

<210> 31
<211> 243
<212> DNA
<213> Artificial Sequence

<220>
<223> truncation mutant FGF18(dc108)

<400> 31

```
atggccgagg agaatgtgga cttccgcatc cacgtggaga accagacgcg ggctcgagat      60
gatgtgagtc ggaagcagct gcgcttgtac cagctctata gcaggaccag tgggaagcac     120
attcaagtcc tgggccgtag gatcagtgcc cgtggcgagg acggggacaa gtatgcccag     180
ctcctagtgg agacagatac cttcgggagt caagtccgga tcaagggcaa ggagacagaa     240
tag                                                                   243
```

<210> 32
<211> 210
<212> DNA
<213> Artificial Sequence

<220>
<223> truncation mutant FGF18(dc113)

<400> 32

```
atggccgagg agaatgtgga cttccgcatc cacgtggaga accagacgcg ggctcgagat      60
gatgtgagtc ggaagcagct gcgcttgtac cagctctata gcaggaccag tgggaagcac     120
attcaagtcc tgggccgtag gatcagtgcc cgtggcgagg acggggacaa gtatgcccag     180
ctcctagtgg agacagatac cttcgggtga                                      210
```

<210> 33
<211> 174

<212> DNA
<213> Artificial Sequence

<220>
<223> truncation mutant FGF18(dc125)

<400> 33

```
atggccgagg agaatgtgga cttccgcatc cacgtggaga accagacgcg ggctcgagat      60

gatgtgagtc ggaagcagct gcgcttgtac cagctctata gcaggaccag tgggaagcac     120

attcaagtcc tgggccgtag gatcagtgcc cgtggcgagg acggggacaa gtag           174
```

<210> 34
<211> 157
<212> PRT
<213> Artificial Sequence

<220>
<223> truncation mutant FGF18(dc25)

<400> 34

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys Tyr Ala Gln Leu Leu Val Glu
    50                  55                  60

Thr Asp Thr Phe Gly Ser Gln Val Arg Ile Lys Gly Lys Glu Thr Glu
65                  70                  75                  80

Phe Tyr Leu Cys Met Asn Arg Lys Gly Lys Leu Val Gly Lys Pro Asp
                85                  90                  95

Gly Thr Ser Lys Glu Cys Val Phe Ile Glu Lys Val Leu Glu Asn Asn
            100                 105                 110

Tyr Thr Ala Leu Met Ser Ala Lys Tyr Ser Gly Trp Tyr Val Gly Phe
        115                 120                 125

Thr Lys Lys Gly Arg Pro Arg Lys Gly Pro Lys Thr Arg Glu Asn Gln
    130                 135                 140

Gln Asp Val His Phe Met Lys Arg Tyr Pro Lys Gly Gln
145                 150                 155
```

<210> 35
<211> 139
<212> PRT
<213> Artificial Sequence

<220>
<223> truncation mutant FGF18(dc43)

<400> 35

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys Tyr Ala Gln Leu Leu Val Glu
    50                  55                  60

Thr Asp Thr Phe Gly Ser Gln Val Arg Ile Lys Gly Lys Glu Thr Glu
65                  70                  75                  80

Phe Tyr Leu Cys Met Asn Arg Lys Gly Lys Leu Val Gly Lys Pro Asp
                85                  90                  95

Gly Thr Ser Lys Glu Cys Val Phe Ile Glu Lys Val Leu Glu Asn Asn
            100                 105                 110

Tyr Thr Ala Leu Met Ser Ala Lys Tyr Ser Gly Trp Tyr Val Gly Phe
        115                 120                 125

Thr Lys Lys Gly Arg Pro Arg Lys Gly Pro Lys
    130                 135
```

<210> 36
<211> 125
<212> PRT
<213> Artificial Sequence

<220>
<223> truncation mutant FGF18(dc57)

<400> 36

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys Tyr Ala Gln Leu Leu Val Glu
    50                  55                  60

Thr Asp Thr Phe Gly Ser Gln Val Arg Ile Lys Gly Lys Glu Thr Glu
65                  70                  75                  80

Phe Tyr Leu Cys Met Asn Arg Lys Gly Lys Leu Val Gly Lys Pro Asp
                85                  90                  95

Gly Thr Ser Lys Glu Cys Val Phe Ile Glu Lys Val Leu Glu Asn Asn
            100                 105                 110

Tyr Thr Ala Leu Met Ser Ala Lys Tyr Ser Gly Trp Tyr
        115                 120                 125
```

<210> 37
<211> 115
<212> PRT
<213> Artificial Sequence

<220>
<223> truncation mutant FGF18(dc67)

<400> 37

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys Tyr Ala Gln Leu Leu Val Glu
    50                  55                  60
```

Thr Asp Thr Phe Gly Ser Gln Val Arg Ile Lys Gly Lys Glu Thr Glu
65 70 75 80

Phe Tyr Leu Cys Met Asn Arg Lys Gly Lys Leu Val Gly Lys Pro Asp
85 90 95

Gly Thr Ser Lys Glu Cys Val Phe Ile Glu Lys Val Leu Glu Asn Asn
100 105 110

Tyr Thr Ala
115

<210> 38
<211> 100
<212> PRT
<213> Artificial Sequence

<220>
<223> truncation mutant FGF18(dc82)

<400> 38

Met Ala Glu Glu Asn Val Asp Phe Arg Ile His Val Glu Asn Gln Thr
1 5 10 15

Arg Ala Arg Asp Asp Val Ser Arg Lys Gln Leu Arg Leu Tyr Gln Leu
20 25 30

Tyr Ser Arg Thr Ser Gly Lys His Ile Gln Val Leu Gly Arg Arg Ile
35 40 45

Ser Ala Arg Gly Glu Asp Gly Asp Lys Tyr Ala Gln Leu Leu Val Glu
50 55 60

Thr Asp Thr Phe Gly Ser Gln Val Arg Ile Lys Gly Lys Glu Thr Glu
65 70 75 80

Phe Tyr Leu Cys Met Asn Arg Lys Gly Lys Leu Val Gly Lys Pro Asp
85 90 95

Gly Thr Ser Lys
100

<210> 39
<211> 88
<212> PRT
<213> Artificial Sequence

<220>
<223> truncation mutant FGF18(dc94)

<400> 39

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys Tyr Ala Gln Leu Leu Val Glu
    50                  55                  60

Thr Asp Thr Phe Gly Ser Gln Val Arg Ile Lys Gly Lys Glu Thr Glu
65                  70                  75                  80

Phe Tyr Leu Cys Met Asn Arg Lys
                85
```

<210> 40
<211> 74
<212> PRT
<213> Artificial Sequence

<220>
<223> truncation mutant FGF18(dc108)

<400> 40

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys Tyr Ala Gln Leu Leu Val Glu
    50                  55                  60

Thr Asp Thr Phe Gly Ser Gln Val Arg Ile
65                  70
```

<210> 41
<211> 69
<212> PRT
<213> Artificial Sequence

<220>
<223> truncation mutant FGF18(dc113)

<400> 41

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys Tyr Ala Gln Leu Leu Val Glu
    50                  55                  60

Thr Asp Thr Phe Gly
65
```

<210> 42
<211> 57
<212> PRT
<213> Artificial Sequence

<220>
<223> truncation mutant FGF18(dc125)

<400> 42

```
Met Ala Glu Glu Asn Val Asp Phe Arg Ile His Val Glu Asn Gln Thr
1               5                   10                  15

Arg Ala Arg Asp Asp Val Ser Arg Lys Gln Leu Arg Leu Tyr Gln Leu
            20                  25                  30

Tyr Ser Arg Thr Ser Gly Lys His Ile Gln Val Leu Gly Arg Arg Ile
        35                  40                  45

Ser Ala Arg Gly Glu Asp Gly Asp Lys
    50                  55
```

## Description

TECHNICAL FIELD

The present invention relates to a hair growth promoting agent, hair regrowth promoting agent or therapeutic for alopecia comprising a substance which inhibits the growth of hair follicles (synonymous with hair roots), and a method of screening for such substances.

BACKGROUND ART

It is known that a variety of polypeptide growth factors, including various members of the fibroblast growth factor (hereinafter, referred to as "FGF") family, are expressed in skin tissue. In both mouse and human, FGFs are encoded by 22 distinct genes (Non-Patent Document 1). Among them, FGF1, FGF2, FGF5, FGF7, FGF10, FGF13 and FGF22 are reported to be expressed in dermal cells and hair follicular cells to regulate hair growth and skin regeneration (see Non-Patent Documents 2-17 and Patent Document 1).

Non-Patent Document 2-17 and other documents suggest that FGF plays an important role in the growth and differentiation of cutaneous cells. However, it is still unknown as to how the FGF group is involved in the effect of promoting the growth of hair follicles and the resultant hair growth promoting effect and hair regrowth promoting effect.

Under the above-described circumstances, the present inventors found that single administration of FGF18 to mouse skin with hair folliclles in telogen phase (resting phase) induces hair regrowth, and reported that FGF18 is a substance which induces onset of anagen phase (growth phase) in follicles to result in promotion of hair growth (Non-Patent Document 19 and Patent Document 2).


Non-Patent Document 1: Ornitz DM, Itoh N: Fibroblast growth factors. Genome Biol2: REVIE WS3005, 2001

Non-Patent Document 2: du Cros DL: Fibroblast growth factor and epidermal growth factor in hair development. J Invest Dermatol 101: 106S-113S. 1993 .

Non-Patent Document 3: du Cros DL, Isaacs K, Moore GP: Distribution of acidic and basicfibr oblast growth factorsin ovine skin during follicle morphogenesis. J Cell Sci 105: 667-674,1993

Non-Patent Document 4: Herbert JM, Rosenquist T, Gotz J, Martin GR: FGF5 as a regulator of the hair growth cycle: Evidence from targeted and spontaneous mutations. Cell78: 1017-1025, 1994

Non-Patent Document 5: Danilenko DM, Ring BD, Yanagihara D, Benson W, Wiemann B, StarnesCO, Pierce GF: Keratinocyte growth factor is an important endogenous mediator of hair follicle growth, development, and differentiation. American J Pathol147: 145-154, 1995

Non-Patent Document 6: Marchese C, Chedid M, Dirsch OR, et al: Modulation of keratinocyte growth factor and its receptor in reepithelializing human skin. J Exp Med182: 1369-1376, 1995

Non-Patent Document 7: Guo L, Degenstein L, Fuchs E: keratinocyte growth factor is require dfor hair development but not for wound healing. Genes Dev 10: 165-175, 1996

Non-Patent Document 8: Rosenquist TA, Martin GR: Fibroblast growth factor signalling in the hair growth cycle: Expression of the fibroblast growth factor receptor and ligand genes in the murine hair follicle. Developmental Dynamics 205:379-386, 1996

Non-Patent Document 9: Petho-Schramm A, Muller HJ, Paus R: FGF5 and the murine hair cycle. Arch Dermatol Res 288: 264-266, 1996

Non-Patent Document 10: Mitsui S, Ohuchi A, Hotta M, Tsuboi R, Ogawa H: Genes for a range of growth factors and cyclin-dependent kinase inhibitors are expressed by isolated human hair follicles. Br J Dermatol 137:693-698, 1997

Non-Patent Document 11: Ortega S, Ittmann M, Tsang SH, Ehrlich M, Basilico C: Neuronaldefects and delayed wound healing in mice lacking fibroblast growth factor-2. Proc Natl Acad Sci USA 95: 5672-5677, 1998

Non-Patent Document 12: Suzuki S, Kato T, Takimoto H, et al: Localization of rat FGF-5 protein in skin macrophage-like cells and FGF-5S protein in hair follicle: Possible involvement of two Fgf-5 gene products in hair growth cycleregulation. J Invest Dermatol 111: 963-972, 1998

Non-Patent Document 13: Suzuki S, Ota Y, Ozawa K, Imamura T: Dual-mode regulation of hair growth cycle by two Fgf-5 gene products. J Invest Dermatol 114: 456-463, 2000

Non-Patent Document 14: Nakatake Y, Hoshikawa M, Asaki T, Kassai Y, Itoh N: Identification of a novel fibroblast growth factor, FGF-22, preferentially expressed in the inner root sheath of the hair follicle. Biochem Biophys Acta 1517: 460-463, 2001

Non-Patent Document 15: Stenn KS, Paus R: Controls of hair follicle cycling. Physiol Rev81: 449-494, 2001

Non-Patent Document 16: Beyer TA, Werner S, Dickson C, Grose R: Fibroblast growth factor 22 and its potential role during skin development and repair. Exp Cell Res287: 228-236 2003

Non-Patent Document 17: kawano M, Suzuki S, Suzuki M, Oki J, Imamura T: Bulge- and basallayer-specific expression of fibroblast growth factor 13(FHF-2) in mouse skin. J Invest Dermatol 122: 1084-1090, 2004

Non-Patent Document 18: Suzuki S, Ota Y, Ozawa K, Imamura T: Dual-mode regulation of hair growth cycle by two Fgf-5 gene products. J Invest Dermatol 114: 456-463, 2000

Non-Patent Document 19: Kawano M, Komi-Kuramochi A, Asada M, Suzuki M, Oki J, Jiang J, Imamura T: Comprehensive analysis of FGF and FGFR expression in skin: FGF18 is highly expressed in hair follicles and capable of inducing anagen from telogenstage hair follicles. J Invest Dermatol. 2005, 124(5): 877-885

Non-Patent Document 20: ZhangX, Ibrahimi OA, Olsen SK, Umemori H, Mohammadi M, Ornitz DM. Receptor Specificity of the Fibroblast Growth Factor Family: THE COMPLETE MAMMALIAN FGFFAMILY. J Biol Chem 2006, 281(23):15694-15700

Patent Document 1: Japanese Unexamined Patent Publication No. Hei 4-224522

Patent Document 2: Japanese Unexamined Patent Publication No. 2006-83082

DISCLOSURE OF THE INVENTION

PROBLEM FOR SOLUTION BY THE INVENTION

Many people are suffering from thin hair or hair loss. However, there is no hair regrowth treatment agent effective for everyone. It is an object of the present invention to elucidate the mode of action of endogenous factors that regulate hair growth, to determine an endogenous factor that inhibits hair growth, to screen for substances that inhibit the activity or expression of the endogenous factor, and to provide a novel hair growth promoting agent, hair regrowth promoting agent or therapeutic for alopecia.

It is another object of the present invention to provide a screening method for the above-described purpose.

MEANS TO SOLVE THE PROBLEM

As described above, it was already confirmed that single administration of FGF18 to skin with hair follicles in telogen phase promotes hair growth after several weeks of reaction period. Therefore, FGF18 was believed to be a substance that has a hair growth promoting effect.

However, the present inventors have obtained an unexpected, surprising finding when FGF18 was administered to mouse dorsal skin subcutaneously once a day for 8 days after compulsive induction of anagen phase in follicles of the dorsal skin in telogen phase by depilating, so that FGF18 was allowed to persist in hair follicles continuously for observing the state of growth of hair.

Briefly, hair growth progressed well and follicle enlargement occurred at day 9 in the control group which received phosphate-buffered physiological saline under similar conditions whereas hair growth was markedly inhibited the FGF18 administered group.

When administration of FGF18 was stopped after its continuous administration under the same conditions and mice were raised thereafter, growth of hair follicles occurred.

Further, the present inventors performed a detailed functional analysis of FGF18. As a result, it was found that the expression level of a growth factor VEGF (which is considered important for hair follicle growth) increased when dermal papilla cells (which are believed to be a control tower for hair follicle growth) were cultured in the presence of FGF18 and then the medium is deprived of FGF18.

Based on these findings, the previous results of animal experiments can be rather explained as having observed "the effect of decreased FGF18 concentration" that resulted from rapid degradation/invalidation of the singly administered FGF18 protein by the mouse body. Therefore, the present inventors assumed that the continuous presence of FGF18 acts inhibitory against hair growth, and this assumption has been examined experimentally. As a result, this assumption was demonstrated to be correct. Thus, the present invention has been achieved.

Considering that FGF18 is an endogenous factor present in hair follicles by nature, both a substance that inhibits the activity of FGF18 in hair follicles and a substance that inhibits the expression of *FGF18* can be used as a hair growth promoting agent, hair regrowth promoting agent or therapeutic for alopecia.

Substances which inhibit the expression of *FGF18* may be screened for by monitoring whether a test substance promotes the expression of *FGF18* or not in a cultured animal cell or an experimental animal.

By developing the above-described finding further, the present inventors assumed that a substance which works as an antagonist to FGF18 by inhibiting the binding of FGF18 to FGF receptors would inhibit the activity of FGF18 and thus such a substance would have a hair growth promoting effect. Further, the present inventors assumed that an antibody which binds to FGF18 to thereby inhibit the binding of FGF18 to FGF receptors would inhibit the activity of FGF18 and thus such an antibody would have a hair growth promoting effect. Since FGF18 reacts with at least four FGF receptor subclasses, i.e., FGFR1c, FGFR2c, FGFR3c and FGFR4 (Non-Patent Document 20), the present invertors particularly noted the above reactivity to develop a screening system for substances that inhibited the activity of FGF18.

As a result of extensive searches into substances derived from naturally occurring plants and other natural product-derived substances using FGFR3c and/or FGFR4 receptor as the screening system, the present invertors found several natural product-derived substances which would inhibit the activation of FGFR3c and/or FGFR4 receptor by FGF18. Thus, the present invention relating to the substance that inhibits the activity of FGF18 has also been achieved.

The present invention includes the following inventions.

(1) A method of promoting non-therapeutic hair growth or hair regrowth comprising administering a substance that inhibits the activity of FGF18, wherein said substance that inhibits the activity of FGF18 is: (a) a partial peptide of FGF18; (b) an anti-FGF18 antibody; (c) an expression vector in which a cDNA encoding a partial peptide of FGF18 having an inhibitory activity against the activity of FGF18 has been integrated; or (d) an expression vector in which an siRNA having an inhibitory activity against the expression of FGF18 has been integrated.

(2) Use of a substance that inhibits the activity of FGF18, wherein said substance that inhibits the activity of FGF18 is: (a) a partial peptide of FGF18; (b) an anti-FGF18 antibody; (c) an expression vector in which a cDNA encoding a partial peptide of FGF18 having an inhibitory activity against the activity of FGF18 has been integrated; or (d) an expression vector in which an siRNA having an inhibitory activity against the expression of FGF18 has been integrated, for promoting non-therapeutic hair growth or hair regrowth.

(3) A substance that inhibits the activity of FGF18, wherein said substance that inhibits the activity of FGF18 is: (a) a partial peptide of FGF18; (b) an anti-FGF18 antibody; (c) an expression vector in which a cDNA encoding a partial peptide of FGF18 having an inhibitory activity against the activity of FGF18 has been integrated; or (d) an expression vector in which an siRNA having an inhibitory activity against the expression of FGF18 has been integrated, for use in a method of treating alopecia.

(4) A hair growth promoting agent, hair regrowth promoting agent or therapeutic for alopecia, comprising as an active ingredient(s) a substance that inhibits the activity of FGF18, wherein said substance that inhibits the activity of FGF18 is: (a) a partial peptide of FGF18; (b) an anti-FGF18 antibody; (c) an expression vector in which a cDNA encoding a partial peptide of FGF18 having an inhibitory activity against the activity of FGF18 has been integrated; or (d) an expression vector in which an siRNA having an inhibitory activity against the expression of FGF18 has been integrated.

(5) The hair growth promoting agent, hair regrowth promoting agent or therapeutic for alopecia according to (4), wherein the substance that inhibits the activity of FGF18 is a partial peptide of FGF18.

(6) The hair growth promoting agent, hair regrowth promoting agent or therapeutic for alopecia according to (4), wherein the substance that inhibits the activity of FGF18 is an anti-FGF18 antibody.

(7) The hair growth promoting agent, hair regrowth promoting agent or therapeutic for alopecia according to (4), wherein the substance that inhibits the activity of FGF18 is an expression vector in which a cDNA encoding a partial peptide of FGF18 having an inhibitory activity against the activity of FGF18 has been integrated.

(8) The hair growth promoting agent, hair regrowth promoting agent or therapeutic for alopecia according to (4), wherein the substance that inhibits the expression of FGF18 is an expression vector in which an siRNA having an inhibitory activity against the expression of FGF18 has been integrated.

(9) The hair growth promoting agent, hair regrowth promoting agent or therapeutic for alopecia according to any one of (4) to (8), further comprising an other hair growth promoting agent, hair regrowth promoting agent or therapeutic for alopecia, respectively.

(10) An *in vitro* method of screening for the substance that inhibits the activity of FGF18 according to any one of (4) to (6) to thereby obtain candidates for the hair growth promoting agent, hair regrowth promoting agent or therapeutic for alopecia, the method comprising the following steps (a) to (c): (a) compulsively expressing at least one FGF receptor gene selected from *FGFR1c, FGFR2c, FGFR3c* and *FGFR4* on the surface of a cell by means of genetic engineering and culturing the cell, (b) bringing, together with FGF18, a test substance into contact with the cell system obtained in step (a) having the FGF receptor on cell surfaces; and (c) selecting those test substances which exhibited an inhibitory activity against the cell growth promoting activity of FGF18 in step (b).

(11) The method of (10), wherein the FGF receptor is FGFR3c.

(12) The method of (10), wherein the FGF receptor is FGFR4.

(13) The method of any one of (10) to (12), wherein the cell on whose surface the FGF receptor of (8) is compulsively expressed is mouse IL-3-dependent Ba/F3 cell strain.

(14) A method of screening for the substance that inhibits the expression of FGF18 according to (4) to thereby obtain candidates for the hair growth promoting agent, hair regrowth promoting agent or therapeutic for alopecia, the method comprising the following steps (a) to (d): (a) preparing a cultured animal cell or a non-human experimental animal capable of expressing *FGF18* to an observable extent, (b) bringing a test substance into contact with the cultured animal cell of (a), or bringing a test substance into contact with or administering the same to the non-human experimental animal of (a); (c) monitoring the expression of *FGF18* in the cultured animal cell or the non-human experimental animal after step (b), and (d) selecting those test substances which have a function of inhibiting the expression of *FGF18.*

(15) The method of (12), wherein the expression of *FGF18* is monitored in step (c) by extracting mRNA from the non-human experimental animal or the cultured animal cell after step (b) and then analyzing the mRNA level of expressed *FGF18*; and those test substances which have a function of inhibiting the expression of *FGF18* are selected in step (d) by selecting systems that exhibited lower levels of *FGF18* mRNA than when the test substance was not allowed to act.

The hair growth promoting agent, hair regrowth promoting agent or therapeutic for alopecia as defined in (4) to (9) above may further comprise other protein growth factors and/or hair growth promoting agents.

Examples of other protein growth factors include epidermal growth factor, platelet-derived growth factor, factors other than FGF18 belonging to the FGF family, transforming growth factor-$\alpha$, transforming growth factor-$\beta$, factors belonging to the transforming growth factor $\beta$ superfamily, insulin-like growth factor-I and insulin-like growth factor-II. The agent or therapeutic of (4) to (9) above may comprise one or several of the above-listed protein growth factors. It should be noted that other protein growth factors are not limited to those listed above.

Examples of other hair growth promoting agents include, but are not limited to, minoxidil, minoxidil analogues, minoxidil derivatives, antiandrogenic agents and a 5$\alpha$-reductase inhibitor finasteride (Propecia). The agent or therapeutic of (4) to (9) above may comprise one or several of the above-listed hair growth promoting agents.

## EFFECT OF THE INVENTION

According to the present invention, it is possible to provide a hair growth promoting agent, hair regrowth promoting agent or therapeutic for alopecia that act by suppressing an endogenous factor that inhibits the growth of hair follicles. With the hair growth promoting agent, hair regrowth promoting agent or therapeutic for alopecia according to the present invention, hair growth, promotion of hair regrowth and treatment of alopecia in bald or thin-haired head can be achieved more effectively.

Further, according to the screening method of the present invention, it is possible to screen for substances that are effective as hair growth promoting agents, hair regrowth promoting agents or therapeutics for alopecia.

## BRIEF DESCRIPTION OF THE DRAWINGS

Fig. 1. shows by two graphs that the mRNA copy numbers of hair follicle growth promoting factors (A: VEGF; B: noggin) produced by dermal papilla cells increase when FGF18 is removed from the cell medium. "Control" represents the results of the case where FGF18 is contained in the medium; "-FGF18" represents the results of the case where FGF18 is removed from the medium.

Fig. 2 is a diagram demonstrating *in vivo* that the growth of hair follicles is inhibited by continuous administration of FGF18. In this Figure, A is a photomicrograph of a skin section from a control mouse that received PBS; and B is a photomicrograph of a skin section from a mouse that received FGF18.

Fig. 3 demonstrates by two graphs that the FGF18's effect of promoting the growth of FGF receptor (FGFR)-expressing cells is inhibited by partial peptides of FGF 18. The results of analysis using FGFR3c-expressing cells (R3c) and FGFR4-expressing cells (R4) are shown.

A: Test sample was added in 1 μg/ml. <Control> Column 1: No sample added, aside from FGF18. <R3c> Column 2: (d4). Column 3: (d16). Column 4: (d18). Column 5: (d22). Column 6: (d37). Column 7: (d48). Column 8: (d77). Column 9: (d95). <R4> Column 10: (d22). Column 11: (d37). Column 12: (d48). Column 13: (d67). Column 14: (d77). Column 15: (d95).

B: Test sample was added in 100 ng/ml. <Control> Column 1: No sample added, aside from FGF18. <R3c> Column 2: (d4). Column 3: (d12). Column 4: (d16). Column 5: (d18). Column 6: (d37). Column 7: (d48). Column 8: (d67). Column 9: (d95). <R4> Column 10: (d37). Column 11: (d67).

Fig. 4 R4/BaF3 Cell Growth Inhibiting Effect of *Momordica charantia* Hot Water Extract

FGFR4/BaF3 cells were cultured in the presence of FGF18 with the addition of *Momordica charantia* hot water extract. Inhibitory effect of *Momordica charantia* hot water extract against the FGFR4/BaF3 cell growth promoting effect of FGF18 is shown for the case where the extract was added at 0.083%, 0.83% and 8.3%.

Fig. 5 *In vivo* Analysis of *Momordica charantia* Hot Water Extract

Ethanol solution of *Momordica charantia* hot water extract (containing 49.5% ethanol and 1% glycerol) was applied to the dorsal skin of C3H/He mice in telogen phase of the hair cycle daily for 11 days (except for day 5 and day 6). The state of hair regrowth in the mouse dorsal skin on days 10, 14, 17, 21 and 24 is shown in hair regrowth scores.

## BEST MODE FOR CARRYING OUT THE INVENTION

Hereinbelow, the present invention will be described in detail.

By applying the present invention, it is possible to provide a novel hair growth promoting agent, hair regrowth promoting agent and therapeutic for alopecia. These agents and therapeutics share the common feature that they inhibit the activity or expression of FGF18 and that they utilize the action of sustained high concentrations of FGF18 for inhibiting the growth of hair follicles (sometimes called "hair roots").

The hair follicle is an organ that produces hair. The hair follicle growth cycle consists of a growth phase (anagen), a recessing phase (catagen) and a resting phase (telogen) which follows the recessing phase. After the resting phase, the growth phase begins. Generally, in mouse experimental systems, anagen phase is a period from day 1 to day 19 after depilation; and catagen phase is a period from day 20 to day 21 after depilation. It is also known that the hair follicle cycle enters telogen phase at day 21-22 after depilation. During the anagen phase, the growth (elongation) of new hair is activated. Simultaneously, the growth of hair follicles is activated inside the skin, and the bottom part of hair follicles reaches the vicinity of the skin bottom. On the other hand, during the telogen phase, hair follicles stay shallow in the skin in small sizes. Further, the thickness of the skin is completely different between anagen phase and telogen phase. In mice with a colored body hair, melanin is synthesized at the beginning of the anagen phase and a blue skin is visible. Therefore, it is also possible to evaluate the progress of hair follicle growth cycle by observing the blue color from the outside of the skin. Further, when the skin is dissected during the anagen phase and observed from the reverse side, the reverse side of the skin can be seen black because hair follicles with abundant melanin are standing in lines at a high density. To the contrary, during the telogen phase, the reverse side of the skin can be seen remaining white. For example, in mouse experimental systems, the entire dorsal hair of 7 to 8 week-old mice is in telogen phase; by depilating the grown hair, the hair cycle is synchronized and anagen phase begins.

### 1. Substances that Inhibit the Activity of FGF 18

The substance that inhibits the activity of FGF18 and which is contained in the novel hair growth promoting agent, hair regrowth promoting agent or therapeutic for alopecia according to the present invention may, for example, be a partial fragment of FGF18.

FGF18 is synthesized in the cytoplasm of FGF18 producing cells as a polypeptide of 207 amino acids in human and mouse. When this polypeptide is secreted to the outside of cells, its N-terminal signal peptide is cleaved to generate a secreted polypeptide of 181 amino acids which exerts a physiological action. This polypeptide reacts with at least four out of the seven FGF receptor subclasses (FGFR1c, FGFR1b, FGFR2c, FGFR2b, FGFR3c, FGFR3b and FGFR4) and they are FGFR1c, FGFR2c, FGFR3c and FGFR4.

The term "FGF18" as used in the present invention refers not only to human-derived FGF18. Other FGF18 polypeptides derived from, for example, other mammals may also be used. Specific examples of other mammals include, but are not limited to, mouse, rat, chicken, turkey, cattle, pig, sheep and rabbit. For example, it is possible to isolate a gene encoding FGF18 from a non-human mammal by designing a probe based on the nucleotide sequence of a human-derived FGF18 as shown in SEQ ID NO: 1 and using the probe according to conventional methods.

The nucleotide sequence and the amino acid sequence of the signal sequence-deleted human-derived FGF18 are shown in SEQ ID NOS: 1 and 2, respectively. The nucleotide sequence and the amino acid sequence of the signal sequence-deleted mouse-derived FGF18 are shown in SEQ ID NOS: 3 and 14, respectively. As comparison of the amino acid sequences as shown in SEQ ID NOS: 2 and 14 reveals, FGF18 polypeptides have a very high homology in mammals. Thus, it is understood that the function of FGF18 is almost the same among mammals.

Generally, the term "antagonist" refers to a substance that binds to a receptor but, unlike a biological substance (ligand) that stimulates the receptor, does not provoke a biological response or provokes only a relatively weak biological response; alternatively, it is a substance that inhibits the binding between the receptor and its endogenous binding partner to thereby block or dampen biological responses. Since FGF18 reacts with at least four FGF receptor subclasses, FGFR1c, FGFR2c, FGFR3c and FGFR4, among the seven subclasses as described above, the term "FGF18 antagonist effect" as used herein means the effect of a substance which blocks or dampens the response by FGF18 in cells that express any of the following receptors: FGFR1c, FGFR2c, FGFR3c and FGFR4. An FGF18 antagonist inhibits the binding of FGF18 to its receptor, which suppresses the hair growth inhibiting effect resulting from FGF18. Consequently, it can be said that the FGF18 antagonist has a hair growth promoting effect.

With respect to partial peptides of FGF18 having an EGF18 antagonist effect, take human FGF18 as an example; the region spanning from positions 32 to 151 of the amino acid sequence shown in SEQ ID NO: 2 is a region called the core domain which is highly common to the FGF family, and a three-dimensional structure that is solely constructed of this region is believed to have a basic ability to bind to the receptor but not a complete activity; hence, partial peptides comprising the amino acid sequence spanning the region corresponding to the core domain are believed to have an FGF18 antagonist effect. If FGF18 partial peptides comprising the amino acid sequence corresponding to the core domain have a sufficient ability to bind to receptors such as FGFR4 but do not have a sufficient length to provoke FGF18 response, such partial peptides have an EGF18 antagonist effect.

Actually, according to the results confirmed in experiments and shown in Fig. 3, a partial peptide of FGF18 where 16 amino acids from the N-terminus (excluding the methionine residue introduced for initiation of translation) have been deleted has the antagonist effect. It can be said that preferably 22, more preferably 77, most preferably 95 amino acids-deleted partial peptides have a stronger FGF18 antagonist effect. This means that partial peptides in which up to 95 amino acids (closer to the N-terminus) have been deleted do not lose their receptor binding ability but rather have a stronger antagonist effect. Thus, it is evident that the entire sequence of the above-described core domain starting from position 32 in the N-terminal sequence is not essential for the binding to FGF receptors. Even if more N-terminal amino acids are deleted from FGF18, the resultant partial peptide can be said to be an FGF18 antagonist. From these results, it is obvious that partial peptides in which 1st to 31st amino acids counted from the C-terminus of FGF18 (the 3 1 st corresponding to the C-terminus of the core sequence) have been deleted will have an FGF18 antagonist effect. What is more, it is fully expected that partial peptides with a larger C-terminal amino acid deletion would not loose the receptor binding ability completely, so partial peptides with a deletion of 31 or more (e.g., 43, 57, 67, 82, 94, 108, 113, 125, etc.) amino acids from the C-terminus are believed to have a strong FGF18 antagonist effect. Needless to say, the antagonist effect is retained even if N-terminal and C-terminal amino acids are deleted simultaneously. Therefore, it can be said that not only partial peptides comprising the amino acid sequence from positions 32 to 151 but also partial peptides comprising the amino acid sequence preferably from positions 77 to 151, more preferably from positions 95 to 151 have the FGF18 antagonist effect.

Antibodies that bind to FGF18 or FGF18 reactive receptors (i.e., anti-FGF18 antibody or anti-FGFR3c antibody, anti-FGFR4 antibody, etc.) also inhibit the binding of FGF18 to its receptors. Thus, these antibodies inhibit the hair follicle growth inhibiting effect of FGF 18.

Thus, in the present invention, substances that inhibit the receptor-mediated effect of FGF18 (such as substances that inhibit the binding of FGF 18 to its receptors, including the above-described FGF18 antagonists) are referred to as the "substance that inhibits the activity of FGF18".

By applying the screening method described in subsection 2 below, substances having an inhibitory effect against FGF18 activity can be obtained easily. In Examples of the present invention, a *Momordica charantia* extract having an inhibitory effect against FGF18 activity was selected by the screening method, and it was confirmed that the *Momordica charantia* extract inhibits the hair follicle growth inhibitory effect of FGF18 and has a hair growth effect.

When the substance that inhibits the activity of FGF18 is a peptide, protein or glycoprotein, the substance may be of course administered in the form of a preparation containing such a peptide or the like. Alternatively, that substance may be administered as an expression vector in which a DNA encoding the peptide or the like has been integrated. For example, a preparation whose active ingredient is an expression vector in which an FGF18 partial peptide-encoding DNA has been integrated may be used as a hair growth promoting agent, hair regrowth promoting agent or therapeutic for alopecia.

These substances that inhibit the activity of FGF18 may be used alone or in combination as a hair growth promoting agent, hair regrowth promoting agent or therapeutic for alopecia.

2. Screening Method for Substances that Inhibit the Activity of FGF 18

An in vitro method of screening for substances that inhibit the activity of FGF 18 to give candidates for the hair growth promoting agent, hair regrowth promoting agent or therapeutic for alopecia comprises specifically the following steps (a) to (c).

(a) compulsively expressing at least one FGF receptor gene selected from *FGFR1c, FGFR2c, FGFR3c* and *FGFR4* on the surface of a cell by means of genetic engineering and culturing the cell,
(b) bringing, together with FGF18, a test substance into contact with the cell system obtained in step (a) having the FGF receptor on cell surfaces; and
(c) selecting those test substances which exhibited an inhibitory activity against the cell growth promoting activity of FGF18 in step (b).

As the FGF receptor gene, at least one of the FGF receptor genes *FGFR1c, FGFR2c, FGFR3c* and *FGFR4* is used; it has been confirmed that FGF18 binds to these receptors and that FGF18 exerts cell growth effect upon cells having these receptors on their surfaces. Preferably, *FGFR3c* or *FGFR4* is used. To bring a test substance into contact with a cell in step (b), it is typically directly added to the cell culture broth, but in a particular case where the test substance is a protein, a gene encoding the test substance can be transferred into an FGF receptor-expressing cell.

The cell to be used for compulsive expression of an FGF receptor on its surface may be any cell as long as it can be cultured. Preferably, mouse IL-3-dependent Ba/F3 cell strain is used.

In the screening, cells are cultured for 48 hr or more (e.g., about 72 hr) and then those substances which decreased the proliferation capacity of FGF receptor expressing cells by 5% or more, preferably by 10% or more, compared to the addition of FGF18 alone may be selected.

The parent cell with no FGF receptor gene transferred thereinto may be used in control test. It is preferable to provide a step in which the same procedure as in step (b) is performed, except that FGF18 added together with a test substance is replaced by IL-3, to confirm that the test substance does not inhibit the cell growth promoting activity of IL-3 in the parent cell.

3. Substances that Inhibit the Expression of FGF18 and Screening Method for such Substances

Since FGF18 is an endogenous factor present in hair follicles, a substance that inhibits the transcription and translation of *FGF18* in hair follicle cells (i.e., substance that decreases the expression of *FGF18*) is also capable of decreasing the concentration of FGF18 in hair follicle cells to thereby induce the hair growth promoting effect of FGF18. Thus, such a substance has a hair growth promoting effect and a hair regrowth promoting effect.

Such substances that inhibit the expression of *FGF18* may be designed as an siRNA against *FGF18* or its expression vector by known methods. The activity of such substances may be confirmed by monitoring whether or not a test substance inhibits the expression of *FGF18* in cultured animal cells or experimental animals.

Alternatively, such substances that inhibit the expression of *FGF18* may be screened for by monitoring whether or not a test substance inhibits the expression of *FGF18* in cultured animal cells or experimental animals.

Specifically, first, cultured animal cells or non-human experimental animals capable of expressing *FGF18* to an observable extent are prepared. Then, a test substance is brought into contact with or administered to the experimental animal, or a test substance is brought into contact with the cultured animal cell. Experimental animals refer to non-human animals such as mouse, rat, chicken, turkey, cattle, pig, sheep and rabbit. Examples of test substances include, but are not limited to, plant extracts, peptides, proteins, nonpeptidic compounds, low molecular weight compounds, synthetic compounds, fermentation products, cell extracts and animal tissue extracts. These substances may be either novel substances or known substances. To bring it into contact with a cell or experimental animal, the test substance is typically directly added to the cell culture broth or administered to the animal but in a particular case where the test substance is a protein, a gene encoding the test substance can be transferred into an FGF receptor-expressing cell.

Subsequently, the expression of *FGF18* in the cultured animal cell or experimental animal is monitored. The expression of *FGF18* in the cultured animal cell or experimental animal may be monitored, for example, by analysis with conventional methods such as ELISA using FGF18 antibody or by analyzing the *FGF18* mRNA level in the cell or experimental animal by quantitative reverse transcription PCR or Northern blotting.

If, as a result of analysis by any of the above-listed methods, the expression level of *FGF18* in the cultured animal cell or experimental animal cultured in the presence of a test substance is found to be lower than that level in the animal cell cultured in the absence of the test substance, the test substance may be judged to have a potential function of hair growth promotion or hair regrowth. Specifically, if the mRNA level decreased to 0.8-fold or less, preferably to 0.7-fold or less, more preferably to 0.5-fold or less, compared to the level for the case whether the test substance was not allowed to act, the latter can positively be regarded as a substance that inhibits the expression of FGF18. The expression levels of *FGF18* mRNA in cultured keratinocytes, cultured dermal cells or cultured dermal papilla cells vary considerably depending on culture conditions or the type of the cells. So they may be measured individually by the above-mentioned methods or the like and those test substances that 0.8-fold or less decreases in measured values may be used as a criterion for screening.

The FGF18 expression inhibiting substance screened through the above-described steps may be used either alone or in combination as a hair growth promoting agent, hair regrowth promoting agent or therapeutic for alopecia.

4. Hair Growth Promoting Agent, Hair Regrowth Promoting Agent or Therapeutic for Alopecia.

The substances described in above subsections "1. Substances that Inhibit the Activity of FGF18" and/or "3. Substances that Inhibit the Expression of FGF18" are formulated into preparations adapted for skin application (e.g., solutions, creams, ointments, gels, lotions, shampoos or aerosols) and are supplied as hair growth promoting agents, hair regrowth promoting agents or therapeutics for alopecia.

In particular, the hair growth promoting agent, hair regrowth promoting agent or therapeutic for alopecia is administered in the form of a pharmaceutical composition comprising a substance that inhibits the activity of FGF18 and/or a substance that inhibits the expression of FGF18 together with a pharmacologically acceptable carrier adapted for local application. The hair growth promoting agent, hair regrowth promoting agent or therapeutic for alopecia comprising a substance that inhibits the activity of FGF18 and/or a substance that inhibits the expression of FGF18 contains the active compound(s) in a pharmacologically acceptable carrier usually at about 0.01 to about 100 $\mu g/day/cm^2$, preferably about 0.1 to about 10 $\mu g/day/cm^2$. This means that the concentration of FGF18 is usually about 0.01 to about 100 $\mu g/day/cm^2$, preferably about 0.1 to about 10 $\mu g/day/cm^2$ of the active compound in a pharmacologically acceptable carrier.

Further, the pharmacologically acceptable carrier adapted for local application is not particularly limited. Specific examples include, but are not limited to, ointments such as hydrophilic vaseline or polyethylene glycol ointment; pastes such as gum (e.g., xanthan gum); solutions such as alcoholic, aqueous or buffer solution; gels such as aluminum hydroxide or sodium alginate gel; albumins such as human or animal albumin; collagens such as human or animal collagen; celluloses such as alkyl cellulose, hydroxyalkyl cellulose or alkylhydroxyalkyl cellulose; methyl cellulose, hydroxyethyl cellulose, carboxymethyl cellulose, hydroxypropylmethyl cellulose and hydroxypropyl cellulose; polymers such as Pluronic™ polyol as exemplified by Pluronic F-127; tetronics such as Tetronic 1508; and alginates such as sodium alginate.

As the substance that inhibits the activity of FGF18 according to the present invention, an expression vector may be used in which a DNA encoding an FGF18 activity inhibitory protein or peptide (such as a partial peptide of FGF18) has been integrated. In that case, the expression vector may be supplied in such a form that it is used in a conventional gene therapy. The expression vector is provided with sequences such as promoter to drive the expression of a partial peptide of FGF18 or the like in animal cells, but is not particularly limited: Examples of expression vectors which may be used in the present invention include, but are not limited to, plasmid vectors and virus vectors.

The hair growth promoting agent, hair regrowth promoting agent or therapeutic for alopecia according to the present invention may comprise a substance that inhibits the expression of FGF18 as an active ingredient. Briefly, the hair growth promoting agent, hair regrowth promoting agent or therapeutic for alopecia according to the present invention may be supplied as in gene therapy using a vector for expression of siRNA against FGF18. The expression vector is provided with sequences such as promoter to drive the expression of siRNA in animal cells, but is not particularly limited. Examples of expression vectors which may be used in the present invention include, but are not limited to, plasmid vectors and virus vectors. It should be noted here that a substance which inhibits the expression of FGF18 is not limited to the vector for expression of siRNA against FGF18.

As a method of introducing the hair growth promoting agent, hair regrowth promoting agent or therapeutic for alopecia for use in gene therapy into cells, both a gene transfer method using a virus vector and a non-viral, gene transfer method [Nikkei Science, 1994 April issue, pp. 20-45; Experimental Medicine Extra Issue 12(15)(1994); Experimental Medicine Separate Volume "Basic Techniques for Gene Therapy", Yodo-sha (1996)] may be used.

As examples of gene transfer method using a virus vector, methods may be mentioned in which a DNA encoding TR4 or mutant TR4 is integrated in DNA or RNA viruses such as retrovirus, adenovirus, adeno-associated virus, herpes virus, vaccinia virus, poxvirus, poliovirus and sinbis virus. Among these, methods using retrovirus, adenovirus, adeno-associated virus or vaccinia virus are especially preferred.

Examples of non-viral gene-transfer methods include a method in which an expression plasmid is directly administered into muscle (DNA vaccine method), the liposome method, lipofection, microinjection, the calcium phosphate method, electroporation and so on. The DNA vaccine method and the liposome method are especially preferred.

5. Application to *in vitro* Hair Regeneration System

It is possible to construct an *in vitro* hair regeneration system in a regenerated skin tissue using a substance that inhibits the activity of FGF18 and/or a substance that inhibits the expression of FGF18. The term "regenerated skin tissue" as used herein means a tissue consisting of various types of skin cells obtainable by culturing isolated skin stem cells. Various types of skin cells are not particularly limited. They include, for example, epithelial cell in the epidermis, cells in the epithelium basal layer, various cells constituting the hair follicle, dermal cells, adipocytes, and so on. Cells used for regenerating a skin tissue may be any of the following cells: heterologous cells, allogeneic cells and autologous cells.

First, there is no established technology that can be used as the method of regulating the differentiation from skin stem cells into various types of skin cells.. Therefore, the method is not particularly limited in the present invention. For example, growth factor receptors that exhibit different expressions at the stage of spontaneous differentiation may be used in such a way that ligand growth factors corresponding to the respective receptors are added into a medium to thereby achieve selective amplification of cells having different directions of differentiation. After selective amplification of the cells having different directions of differentiation, a skin tissue may be prepared.

Since there is no established technology for preparing artificial skin tissues, the method of preparation is not particularly limited in the present invention. Various methods may be used and examples include: a method in which epithelial cells alone are cultured and layered; a method in which the dermal layer is formed with dermis-constituting cells (such as fibroblast cells) and then epithelial cells are over-layered to form an integral sheet; a method in which the surface of the over-layered epithelial cells is exposed to the air to promote the formation of epidermis; or a method in which a layered film formed of a biodegradable component is used instead of the dermal layer. In the present invention, a skin tissue preparation method used in "regenerative therapy" may also be applied; e.g., skin stem cells are isolated from a skin tissue collected from a human, and a new skin tissue is prepared from the resultant skin stem cells. At this time, the new skin tissue may be prepared on the assumption that it is to be returned to the human donor for the purpose of treatment, or on the assumption that it is to be transplanted to a human different from the human donor for the purpose of treatment.

In such a skin tissue preparation method, if a substance that inhibits the activity of FGF18 and/or a substance that inhibits the expression of FGF18 is added to the medium at an appropriate time, it is possible to promote hair follicle growth in the regenerated skin tissue and to thereby promote hair growth or regrowth in the skin tissue. Further, in a skin tissue preparation method, if a substance that inhibits the activity of FGF18 and/or a substance that inhibits the expression of FGF18 is added to the medium at an appropriate time, it is possible to promote the growth of skin cells in the regenerated skin tissue and to thereby increase the volume of the entire skin tissue.

## EXAMPLES

Hereinbelow, the present invention will be described with reference to the following Examples. However, the technical scope of the present invention is not limited to these Examples.

### [EXAMPLE 1]

Inhibitory Effect of FGF18 on the Gene Expression of Hair Follicle Growth Promoting Factor

In this Example, in order to evaluate the function of FGF18 on hair growth, the effect of FGF18 on gene expression was examined in dermal papilla cells which are believed to be a control tower for hair follicle growth.

<Materials and Methods>

Cultured human dermal papilla cells (HFDP; from adult human scalp; Toyobo) were subcultured in HFDP growth medium (20% fetal bovine serum-containing basal medium for dermal papilla cell; Toyobo) and used in experiments within two passages. The cells were treated with trypsin and seeded in collagen-coated dishes (Sumilon; 6 cm in diameter) at $4x10^5$ cells/dish. The cells were maintained at 37°C in HFDP medium. On the next day, the medium was exchanged for HEK medium (EpiLife™, 0.06 mM $CaCl_2$, 10 μg/ml insulin, 0.1 ng/ml hEGF, 0.5 μg/ml hydrocortisone, 0.4% BPE), and then FGF18 was added to the medium. After the cells were cultured for 24 hr, the medium was exchanged for FGF18 free medium in the test sample group ("-FGF18"). The control group ("Control") was cultured continuously in the FGF18-containing medium. Subsequently, the cells were harvested and the mRNA was extracted and purified. The expression levels of mRNA from VEGF (known as a hair follicle growth promoting factor) and noggin (known as a hair growth inhibiting factor) contained in the resultant mRNA were analyzed.

<Results>

The results are shown in Fig. 1. It is believed that dermal papilla cells release various factors to thereby support hair growth. Among them are VEGF, which promotes hair growth, and noggin. In this experiment, the expression level of *VEGF* mRNA in dermal papilla cells increased to 4.8-fold by depriving the medium of FGF18. Further, the expression level of *noggin* mRNA increased to 3.5-fold by depriving the medium of FGF18. From these results, it was confirmed that the production of VEGF in dermal papilla cells which was inhibited in the presence of FGF18 is promoted by elimination of the effect of FGF18. Briefly, it was suggested that FGF18 has a hair growth inhibiting effect and that inhibition of the activity or expression of this FGF18 would act favorably on the growth of hair follicles.

[EXAMPLE 2]

Hair Growth Inhibiting Effect by Continuous Administration of FGF18

In this Example, in order to examine the effect of FGF18 *in vivo* on hair follicle growth, the effect of FGF18 administration was tested in hair follicle anagen phase-induced C3H/HeN mice.

<Materials and Methods>

In order to examine the effect of FGF18 *in vivo* on hair follicle growth, FGF18 protein dissolved in phosphate buffered physiological saline (PBS) was administered to hair follicle anagen phase-induced mice.

Briefly, dorsal hair of 50 day-old C3H/HeN male mice in telogen phase was depilated gently with fingers to thereby induce the start of hair follicle anagen phase. Then, FGF18 solution was injected into the dorsal skin subcutaneously from the vicinity of the tail (1 μg of FGF18 per mouse). The mice were maintained on a diet and water *ad libitum.* Starting from this day, FGF18 solution was injected subcutaneously into the dorsal skin every day at about the same time for consecutive 8 days. The control group received injection of PBS instead of FGF18. Nine days after the initial injection, the mice were euthanized under anesthesia. Full thickness skin samples were excised from the dorsal part and embedded in paraffin. The thus embedded skin samples were sliced into 4 μm thick sections with a microtome, stained with hematoxylin and observed under microscope.

<Results>

The results are shown in Fig. 2. In this Figure, A represents a photomicrograph of a skin section from a control mouse which received PBS; B represents a photomicrograph of a skin section from an FGF18-administered mouse. In A and B, the magnification is the same.

As seen from photomicrograph A, natural growth of hair follicles was observed at day 9 in the PBS-administered mouse. Hair follicles had grown long and reached the lower layer of the skin.

On the other hand, in the FGF18 solution-administered mouse in photomicrograph B, hair follicles are short, suggesting that hair growth is strongly inhibited.

From these results, it was demonstrated that continuous administration of FGF18 inhibits hair growth. Conversely, this suggests that hair follicle growth, if it is being inhibited by continuous presence of endogenous FGF18, can be promoted by inhibiting the activity of FGF18.

[Example 3]

Inhibition of FGF18 Activity by Partial Peptides of FGF18

As FGF18 partial peptides, partial polypeptides were prepared based on SEQ ID NO: 14 which corresponds to the amino acid sequence of mouse FGF18. Briefly, from up to position 4 to up to position 95 amino acids as counted from the N-terminus (excluding the methionine introduced for initiation of translation) were deleted to prepare partial peptides d4-d95 [indicating the number of amino acids deleted from N-terminal (excluding methionine)].

The amino acid sequences (nucleotide sequences) of the individual partial peptides correspond to the following SEQ ID NOS.

d4: SEQ ID NO: 15 (SEQ ID NO: 4)
d12: SEQ ID NO: 16 (SEQ ID NO: 5)

d 16: SEQ ID NO: 17 (SEQ ID NO: 6)
d18: SEQ ID NO: 18 (SEQ ID NO: 7)
d22: SEQ ID NO: 19 (SEQ ID NO: 8)
d37: SEQ ID NO: 20 (SEQ ID NO: 9)
d48: SEQ ID NO: 21 (SEQ ID NO: 10)
d67: SEQ ID NO: 22 (SEQ ID NO: 11)
d77: SEQ ID NO: 23 (SEQ ID NO: 12)
d95: SEQ ID NO: 24 (SEQ ID NO: 13)

FGF18 stimulates the four FGF receptors FGFR1c, FGFR2c, FGFR3c and FGFR4, but the intensity of stimulation varies depending on the receptor. It is believed that summation of stimulations on these receptors results in inhibition of hair growth.

According to teachings disclosed in literature, the FGF receptor genes *FGFR1c, FGFR2c, FGFR3c* and *FGFR4* were respectively introduced by means of genetic engineering into Ba/F3 cell strain (obtained from RIKEN BRC) which is mouse IL-3-dependent proB cell to thereby prepare cells on whose surface FGFR was compulsively expressed (Ornitz, DM., Xu, J., Colvin, JS., McEwen, DG, MacArthur, CA., Coulier, F., Gao, G and Goldfarb, M., 1996. Receptor specificity of the fibroblast growth factor family. J. Biol. Chem. 271(25):15292-15297; Yoneda, A., Asada, M., Oda, Y, Suzuki, M. and Imamura, T, 2000. Engineering of an FGF-proteoglycan fusion protein with heparin-independent, mitogenic activity. Nat. Biotec. 18(6):641-644).

Using these cells, the antagonist activity of test samples [whether or not test samples inhibit the DNA synthesis of FGF18-stimulated cell (this is taken as 100%)] was examined while stimulating each receptor with 30 ng/ml FGF18, in the co-presence of each partial peptide of FGF18 at 1 μg/ml (Fig/ 3A) or at 100 ng/ml (Fig. 3B). Briefly, an activity that inhibits the cell growth stimulating effect of FGF18 was analyzed. The results are shown in Fig. 3. Correspondence between individual columns and test samples is as described below.

(Fig. 3A) Column 1: no sample added, other than FGF 18 (control). Column 2: (d4). Column 3: (d16). Column 4: (d18). Column 5: (d22). Column 6: (d37). Column 7: (d48). Column 8: (d77). Column 9: (d95). Column 10: (d22). Column 11: (d37). Column 12: (d48). Column 13: (d67). Column 14: (d77). Column 15: (d95).

(Fig. 3B) Column 1: no sample added, other than FGF18 (control). Column 2: (d4). Column 3: (d12). Column 4: (d16). Column 5: (d18). Column 6: (d37). Column 7: (d48). Column 8: (d67). Column 9: (d95). Column 10: (d37). Column 11: (d67).

From Fig. 3A, it is seen that test samples such as d16, d22, d37, d48, d77, d95, etc, when tested at the concentration of 1 μg/ml, strongly inhibit the activity of FGF18 on FGFR3c/BaF3 cells. It is also seen that d22, d48, d77, d95, etc. strongly inhibit the activity of FGF18 on FGFR4/BaF3 cells. Other partial peptides also exhibit various inhibitory effects as shown in this Figure.

From Fig. 3B, it is seen that test samples such as d16, d95, etc, when tested at the concentration of 100 ng/ml, strongly inhibit the activity of FGF18 on FGFR3c/BaF3 cells.

By using these partial polypeptides of FGF18 which act as FGF18 activity inhibiting substances, eliminated the hair growth inhibiting effect of FGF18 in the regulation of hair growth can be, thus, achieving promoted hair growth.

Further, partial polypeptides were prepared in the same manner based on SEQ ID NO: 14 which corresponds to the amino acid sequence of mouse FGF18 by deleting amino acids from up to position 25 to up to position 125 counted from the C-terminus. Partial peptides dc25-dc125 (indicating the number of amino acids deleted from C-terminal) were prepared.

The amino acid sequences (nucleotide sequences) of individual partial peptides correspond to the following SEQ ID NOS.

dc25: SEQ ID NO: 34 (SEQ ID NO: 25)
dc43: SEQ ID NO: 35 (SEQ ID NO: 26)
dc57: SEQ ID NO: 36 (SEQ ID NO: 27)
dc67: SEQ ID NO: 37 (SEQ ID NO: 28)
dc82: SEQ ID NO: 38 (SEQ ID NO: 29)
dc94: SEQ ID NO: 39 (SEQ ID NO: 30)
dc108: SEQ ID NO: 40 (SEQ ID NO: 31)
dc113: SEQ ID NO: 41 (SEQ ID NO: 32)
dc125: SEQ ID NO: 42 (SEQ ID NO: 33)

All these partial polypeptide of FGF18 except dc25 have an inhibitory effect against FGF18 activity similar to the effect of the above-listed N-terminal deleted partial peptides. Therefore, these partial polypeptide are capable of eliminating the hair growth inhibiting effect of FGF18 and promoting hair growth.

[EXAMPLE 4]

Screening for FGF18 Activity Inhibiting Substances Using FGFR4 Expressing Cell

The cell on whose surface FGR4 is compulsively expressed (R4/Ba/F3 cell) prepared above was cultured using various plant extracts as test substances in the presence of FGF18. As a positive control, a commercial FGF18 protein was used. The cell count after culturing for a specific time period was determined with Cell Counting Kit-8 (manufactured by Dojindo Laboratories and sold by Wako Pure Chemical Industries) by measuring the coloring at 450 nm which was proportionate to the yield of WST-8 formazan.

As a result, it was found that *Momordica charantia* extract acts as an FGF18 activity inhibiting substance and inhibits the growth of FGFR4/BaF3 cell due to FGF 18.

[EXAMPLE 5]

Cell Growth Inhibiting Activity of the FGF18 Activity Inhibiting Substance identified in Example 4

To 1.5 g of dried *Momordica charantia,* 30 ml of distilled water was added and the resultant mixture was boiled for 15 min. The thus obtained extract was filtered with a filter paper. The filtrate was collected to obtain a *Momordica charantia* hot water extract. To 1.5 g of dried *Momordica charantia,* 30 ml of 70% ethanol aqueous solution was added, followed by soaking extraction at room temperature for 7 days. The thus obtained extract was filtered with a filter paper to give a 70% ethanol extract of *Momordica charantia.*

In the same manner as in Example 4, the activity of the FGF18 activity inhibiting substance was measured using FGFR4/Ba/F3 cell. Specifically, measurement was performed as described below. Briefly, RPMI1640 medium containing 10% FBS and 1% Antibiotic G-418 Sulfate (Promega; V7983) was added to each well of 96-well cell culture plates (50 μl/well). Subsequently, various concentrations of test solutions prepared by diluting samples with water were added (10 μl/well), followed by addition of 50 μl of cell suspension in which $5x10^4$ R4/Ba/F3 cells were suspended in RPMI1640 medium containing 10% FBS and 1% Antibiotic G-418 Sulfate. The resultant mixture was stirred lightly. Further, 10 μl of heparin/10% FBS/1% Antibiotic G-418 Sulfate/RPMI1640 medium (final heparin concentration: 5 μg/ml) and 10 μl of FGF18 (PeproTech; 100-28) solution (final concentration of FGF18: 3 ng/ml) were added thereto. Then, the cell was cultured in a carbon dioxide incubator at 37°C under 5% $CO_2$ for 72 hr. To determine the growth of FGFR4/Ba/F3 cell, 10 μl of Cell Counting Kit-8 (manufactured by Dojindo Laboratories and sold by Wako Pure Chemical Industries)/PBS solution was added to each well after 72-hour culture, followed by culturing for another 3 hrs, and the coloring at 450 nm which was proportionate to the yield of WST-8 formazan was measured.

In the measurement, 10 μl of FGF18 (PeproTech; 100-28) solution (final FGF18 concentration: 3 ng/ml) was used as a positive control. As a negative control, 10 μl of water or ethanol solution (final concentration of ethanol was adjusted to 1% or less) used for preparing test solutions was used. Cell growth inhibition rate (%) was calculated by formula (A) described below.

[Formula (A)]

Cell growth inhibition rate (%) = 100 x {(Absorbance upon addition of FGF18 and water or ethanol solution) - (Absorbance upon addition of FGF18 and test sample)}/ Absorbance upon addition of FGF 18 and water or ethanol solution - Absorbance upon addition of water or ethanol solution)

The results on *Momordica charantia* hot water extract are shown in Fig. 4. When *Momordica charantia* hot water extract with final concentration 8.3% was used as a test sample, the coloring decreased. Thus, it was confirmed that *Momordica charantia* hot water extract inhibits the growth of FGFR4/Ba/F3 cell due to FGF18. On the other hand, the absorbance upon addition of the test sample in the absence of FGF 18 hardly decreased, as compared with the absorbance upon addition of water or ethanol solution in the absence of FGF18. Thus, the cytotoxic effect of the added test sample was hardly observed.

[EXAMPLE 6]

In order to examine the *in vivo* effect of the FGF18 activity inhibiting substance identified in Example 4, a test was performed on C3H/He mice in telogen phase of the hair cycle.

*In vivo* Analysis of the FGF18 Activity Inhibiting Substance identified in Example 4

Fifteen ml of distilled water was added to 1.1 g of dried *Momordica charantia.* The resultant mixture was boiled for 20 min. After the extract returned to room temperature, it was filtered with a filter paper. By adding an equal volume of ethanol to the filtrate, 50% ethanol solution of *Momordica charantia* hot water extract was prepared. This solution was filtered through a 0.45 μm Millex HV sterilization filter (Millipore). Then, glycerol was added to the filtrate to give a concentration of 1% to thereby prepare a test solution. The thus prepared *Momordica charantia* hot water extract (containing 49.5% ethanol and 1 % glycerol) was applied to the dorsal skin of C3H/He mice in telogen phase of the hair cycle. After anesthetizing, dorsal hair of 7 week-old C3H/He male mice was shaved gently with a hair clipper. After shaving, 200 μl of the *Momordica charantia* extract as a test solution (containing 49.5% ethanol and 1 % glycerol) or water-ethanol-glycerol solution (containing 49.5% water, 49.5% ethanol and 1 % glycerol) was applied to the dorsal skin of 5 mice per group (day 0). In a similar manner, application was performed daily for 11 days (excluding day 5 and day 6). The state of hair regrowth in the shaved area in the back of mice was observed with eyes at day 10, day 14, day 17, day 21 and day 24, to thereby give hair regrowth scores. Each state was scored as follows: 1) pigmentation: 1 point; 2) short hair: 2 points; 3) normal hair: 3 points. The ratio of the area of each hair regrowth state to the total shaved area was determined in %. Then, hair regrowth score was calculated by the formula described below. According to this calculation method, the score is 100 when the total shaved area has been recovered to normal hair state.

**Hair regrowth score = [ratio of pigmentation area (%) x 1 + ratio of short hair area (%) x 2 + ratio of normal hair area (%) x 3]/3**

The state of hair regrowth in the shaved area in the back of mice was observed at day 10, day 14, day 17, day 21 and day 24 after the first application. As a result, as shown in Fig. 5, the *Momordica charantia* extract treated group exhibited higher hair regrowth scores, particularly on day 21 and thereafter, than the negative control group (treated with water-ethanol-glycerol solution containing 49.5% water, 49.5% ethanol and 1 % glycerol). Thus, hair regrowth was promoted significantly by *Momordica charantia* extract.

As described so far, it was demonstrated that *Momordica charantia* hot water extract, an FGF18 activity inhibiting substance, has an effect as a hair regrowth promoting agent.

[EXAMPLE 7]

A formulation of a hair shampoo comprising the *Momordica charantia* hot water extract and a method of preparing the shampoo are described below.

A hair shampoo was prepared according to the following formulation and preparation method.

(Formulation)

| Component | Weight % |
|---|---|
| 1. Diluted solution obtained in Example 5 | 0.1 |
| 2. Sodium laurylether sulfate ethanol | 20 |
| 3. Sodium lauryl sulfate | 10 |
| 4. 1,3-Butylene glycol | 1 |
| 5. Flavor | proper quantity |
| 6. Purified water | to make the total 100 |

(Preparation Method)

The components listed above were heated to 80°C, mixed by stirring and then cooled under stirring. Thus, the shampoo was prepared.

[EXAMPLE 8]

A formulation of a hair liquid comprising a *Momordica charantia* hot water extract and a method of preparing the hair liquid are described below.

A hair liquid was prepared according to the following formulation and preparation method.

(Formulation)

| Component | Weight % |
|---|---|
| 1. Diluted solution obtained in Example 5 | 0.1 |
| 2. Ethanol | 40 |
| 3. Glycerol | 1 |
| 4. Flavor | proper quantity |
| 5. Purified water | to make the total 100 |

(Preparation Method)

The components listed above other than purified water were dissolved by stirring and then purified water was added. Thus, the hair liquid was prepared.

[EXAMPLE 9]

A formulation of a hair cream comprising a *Momordica charantia* hot water extract and a method of preparing the hair cream are described below.

A hair cream was prepared according to the following formulation and preparation method.

(Formulation)

| Component | Weight % |
|---|---|
| 1. Diluted solution obtained in Example 5 | 0.1 |
| 2. Liquid paraffin | 40 |
| 3. Vaseline | 1 |
| 4. Cetostearyl alcohol | 1 |
| 5. Methyl polysiloxane | 1 |
| 6. Methyl paraoxybenzoate | 0.2 |
| 7. Propylene glycol | 5 |
| 8. Flavor | proper quantity |
| 9. Purified water | to make the total 100 |

(Preparation Method)

The components listed above were mixed by stirring to thereby prepare the hair cream.

INDUSTRIAL APPLICABILITY

According to the present invention, effective hair growth promoting agents, hair regrowth promoting agents and therapeutics for alopecia are provided. By incorporating these agents, it is possible to provide shampoos and hair liquids with hair growth promoting activity, as well as pharmaceutical compositions for treating alopecia.

## Claims

1. A method of promoting non-therapeutic hair growth or hair regrowth comprising administering a substance that inhibits the activity of FGF18, wherein said substance that inhibits the activity of FGF18 is:

   (a) a partial peptide of FGF18;
   (b) an anti-FGF18 antibody;
   (c) an expression vector in which a cDNA encoding a partial peptide of FGF 18 having an inhibitory activity against the activity of FGF18 has been integrated; or
   (d) an expression vector in which an siRNA having an inhibitory activity against the expression of FGF18 has been integrated.

2. The method of claim 1, wherein said substance that inhibits the activity of FGF18 is used in combination with another hair growth promoting agent or hair regrowth promoting agent.

3. Use of a substance that inhibits the activity of FGF18, wherein said substance that inhibits the activity of FGF 18 is:

(a) a partial peptide of FGF18;
(b) an anti-FGF 18 antibody;
(c) an expression vector in which a cDNA encoding a partial peptide of FGF18 having an inhibitory activity against the activity of FGF18 has been integrated; or
(d) an expression vector in which an siRNA having an inhibitory activity against the expression of FGF18 has been integrated,

for promoting non-therapeutic hair growth or hair regrowth.

**4.** The use of claim 3, wherein said substance that inhibits the activity of FGF18 is used in combination with another hair growth promoting agent or hair regrowth promoting agent.

**5.** A substance that inhibits the activity of FGF18, wherein said substance that inhibits the activity of FGF18 is:

(a) a partial peptide of FGF18;
(b) an anti-FGF18 antibody;
(c) an expression vector in which a cDNA encoding a partial peptide of FGF18 having an inhibitory activity against the activity of FGF18 has been integrated; or
(d) an expression vector in which an siRNA having an inhibitory activity against the expression of FGF18 has been integrated,

for use in a method of treating alopecia.

**6.** The substance for use according to claim 5, wherein said substance that inhibits the activity of FGF18 is used in combination with another hair growth promoting agent or hair regrowth promoting agent.

**7.** The substance for use according to claim 5, wherein said substance is used in combination with another therapeutic for alopecia.

**8.** A hair growth promoting agent, hair regrowth promoting agent or therapeutic for alopecia, comprising as an active ingredient(s) a substance that inhibits the activity of FGF 18, wherein said substance that inhibits the activity of FGF18 is:

(a) a partial peptide of FGF18;
(b) an anti-FGF18 antibody;
(c) an expression vector in which a cDNA encoding a partial peptide of FGF18 having an inhibitory activity against the activity of FGF18 has been integrated; or
(d) an expression vector in which an siRNA having an inhibitory activity against the expression of FGF18 has been integrated.

**9.** The hair growth promoting agent, hair regrowth promoting agent or therapeutic for alopecia according to claim 8, further comprising another hair growth promoting agent, hair regrowth promoting agent or therapeutic for alopecia, respectively.

**10.** An *in vitro* method of screening for a substance that inhibits the activity of FGF18 according to any one of claims 1 to 8 to thereby obtain candidates for the hair growth promoting agent, hair regrowth promoting agent or therapeutic for alopecia, said method comprising the following steps (a) to (c):

(a) compulsively expressing at least one FGF receptor gene selected from *FGFR1c, FGFR2c, FGFR3c* and *FGFR4* on the surface of a cell by means of genetic engineering and culturing the cell,
(b) bringing, together with FGF18, a test substance into contact with the cell system obtained in step (a) having the FGF receptor on cell surfaces; and
(c) selecting those test substances which exhibited an inhibitory activity against the cell growth promoting activity of FGF18 in step (b).

**11.** The method according to claim 10, wherein the FGF receptor is FGFR3c or FGFR4.

**12.** The method according to claim 10 or 11, wherein the cell on whose surface the FGF receptor according to claim 10 is compulsively expressed is mouse IL-3-dependent Ba/F3 cell strain.

13. A method of screening for a substance that inhibits the expression of FGF18 according to any one of claims 1 to 8 to thereby obtain candidates for the hair growth promoting agent, hair regrowth promoting agent or therapeutic for alopecia, said method comprising the following steps (a) to (d):

(a) preparing a cultured animal cell or a non-human experimental animal capable of expressing *FGF18* to an observable extent,
(b) bringing a test substance into contact with the cultured animal cell of (a), or bringing a test substance into contact with or administering the same to the non-human experimental animal of (a),
(c) monitoring the expression of *FGF18* in the cultured animal cell or the non-human experimental animal after step (b), and
(d) selecting those test substances which have a function of inhibiting the expression of *FGF18.*

14. The method according to claim 13, wherein the expression of *FGF18* is monitored in step (c) by extracting mRNA from the non-human experimental animal or the cultured animal cell after step (b) and then analyzing the mRNA level of expressed *FGF18;* and those test substances which have a function of inhibiting the expression of *FGF18* are selected in step (d) by selecting systems that exhibited lower levels of *FGF18* mRNA than when the test substance was not allowed to act.

**Patentansprüche**

1. Verfahren zum Fördern nicht therapeutischen Haarwuchses oder Haarnachwuchses, umfassend das Verabreichen einer Substanz, welche die Aktivität von FGF18 inhibiert, wobei besagte Substanz, welche die Aktivität von FGF18 inhibiert, ist:

(a) ein partielles Peptid von FGF18;
(b) ein Anti-FGF18-Antikörper;
(c) ein Expressionsvektor, in welchen eine cDNA, welche ein partielles Peptid von FGF18, das eine inhibitorische Aktivität gegen die Aktivität von FGF18 hat, kodiert, integriert wurde; oder
(d) ein Expressionsvektor, in welchen eine siRNA, die eine inhibitorische Aktivität gegen die Expression von FGF18 hat, integriert wurde.

2. Verfahren gemäß Anspruch 1, wobei besagte Substanz, welche die Aktivität von FGF18 inhibiert, in Kombination mit einem anderen Haarwuchs-fördernden Mittel oder Haarnachwuchs-fördernden Mittel verwendet wird.

3. Verwendung einer Substanz, welche die Aktivität von FGF18 inhibiert, wobei besagte Substanz, welche die Aktivität von FGF18 inhibiert, ist:

(a) ein partielles Peptid von FGF18;
(b) ein Anti-FGF18-Antikörper;
(c) ein Expressionsvektor, in welchen eine cDNA, welche ein partielles Peptid von FGF18, das eine inhibitorische Aktivität gegen die Aktivität von FGF18 hat, kodiert, integriert wurde; oder
d) ein Expressionsvektor, in welchen eine siRNA, die eine inhibitorische Aktivität gegen die Expression von FGF18 hat, integriert wurde,

zum Fördern nicht-therapeutischen Haarwuchses oder Haarnachwuchses.

4. Verwendung gemäß Anspruch 3, wobei besagte Substanz, welche die Aktivität von FGF18 inhibiert, in Kombination mit einem anderen Haarwuchs-fördernden Mittel oder Haarnachwuchs-fördernden Mittel verwendet wird.

5. Substanz, welche die Aktivität von FGF18 inhibiert, wobei besagte Substanz, welche die Aktivität von FGF18 inhibiert, ist:

(a) ein partielles Peptid von FGF18;
(b) ein Anti-FGF18-Antikörper;
(c) ein Expressionsvektor, in welchen eine cDNA, welche ein partielles Peptid von FGF18, das eine inhibitorische Aktivität gegen die Aktivität von FGF18 hat, kodiert, integriert wurde; oder
(d) ein Expressionsvektor, in welchen eine siRNA, die eine inhibitorische Aktivität gegen die Expression von FGF18 hat, integriert wurde,

zur Verwendung in einem Verfahren zur Behandlung von Alopezie.

**6.** Substanz zur Verwendung gemäß Anspruch 5, wobei besagte Substanz, welche die Aktivität von FGF18 inhibiert, in Kombination mit einem anderen Haarwuchs-fördernden Mittel oder Haarnachwuchs-fördernden Mittel verwendet wird.

**7.** Substanz zur Verwendung gemäß Anspruch 5, wobei besagte Substanz in Kombination mit einem anderen Therapeutikum gegen Alopezie verwendet wird.

**8.** Haarwuchs-förderndes Mittel, Haarnachwuchs-förderndes Mittel oder Therapeutikum gegen Alopezie, umfassend als Wirkstoff(e) eine Substanz, welche die Aktivität von FGF18 inhibiert, wobei besagte Substanz, welche die Aktivität von FGF18 inhibiert, ist:

(a) ein partielles Peptid von FGF18;
(b) ein Anti-FGF18-Antikörper;
(c) ein Expressionsvektor, in welchen eine cDNA, welche ein partielles Peptid von FGF18 das eine inhibitorische Aktivität gegen die Aktivität von FGF18 hat, kodiert, integriert wurde; oder
(d) ein Expressionsvektor, in welchen eine siRNA, die eine inhibitorische Aktivität gegen die Expression von FGF18 hat, integriert wurde.

**9.** Haarwuchs-förderndes Mittel, Haarnachwuchs-förderndes Mittel oder Therapeutikum gegen Alopezie gemäß Anspruch 8, des Weiteren umfassend ein anderes Haarwuchs-förderndes Mittel, Haarnachwuchs-förderndes Mittel oder Therapeutikum gegen Alopezie.

**10.** In-vitro-Verfahren zum Screening auf eine Substanz, welche die Aktivität von FGF18 inhibiert gemäß einem der Ansprüche 1 bis 8, um dadurch Kandidaten für das Haarwuchs-fördernde Mittel, Haarnachwuchs-fördernde Mittel oder Therapeutikum gegen Alopezie zu erhalten, besagtes Verfahren umfasst die folgenden Schritte (a) bis (c):

(a) erzwungenes Exprimieren wenigstens eines FGF18-Rezeptorgens, ausgewählt aus *FGFR1c, FGFR2c, FGFR3c* und *FGFR4,* auf der Oberfläche einer Zelle durch Gentechnologie und Kultivieren der Zelle,
(b) in Kontakt bringen, zusammen mit FGF18, einer Testsubstanz mit dem Zellsystem, welches in Schritt (a) erhalten wurde und den FGF-Rezeptor auf der Zelloberfläche hat; und
(c) Auswählen solcher Testsubstanzen, welche eine inhibitorische Aktivität gegen die Zellwachstums-fördernde Aktivität von FGF18 in Schritt (b) zeigten.

**11.** Verfahren gemäß Anspruch 10, wobei der FGF-Rezeptor *FGFR3c* oder *FGFR4* ist.

**12.** Verfahren gemäß Anspruch 10 oder 11, wobei die Zelle, auf deren Oberfläche der FGF-Rezeptor gemäß Anspruch 10 erzwungener Weise exprimiert wird, ein IL-3-abhängiger Ba/F3-Maus-Zellstamm ist.

**13.** Verfahren zum Screening auf eine substanz, welche die Expression von FGF18 inhibiert, gemäß einem der Ansprüche 1 bis 8, um dadurch Kandidaten für das Haarwuchs-fördernde Mittel, Haarnachwuchs-fördernde Mittel oder Therapeutikum gegen Alopezie zu erhalten, besagtes Verfahren umfasst die folgenden Schritte (a) bis (d):

(a) Herstellen einer kultivierten Tierzelle oder eines nicht-menschlichen Versuchstieres, welche(s) fähig ist, FGF18 in erkennbarem Umfang zu exprimieren,
(b) in Kontakt bringen einer Testsubstanz mit der kultivierten Tierzelle nach (a) oder in Kontakt bringen mit oder Verabreichen derselben an das nicht-menschliche Versuchstier nach (a)
(c) Überwachen der Expression von FGF18 in der kultivierten Tierzelle oder dem nicht-menschlichen Versuchstier nach Schritt (b), und
(d) Auswählen solcher Testsubstanzen, welche die Funktion haben, die Expression von FGF18 zu inhibieren.

**14.** Verfahren gemäß Anspruch 13, wobei die Expression von FGF18 in Schritt (c) durch Extrahieren von mRNA aus dem nicht-menschlichen Versuchstier oder der kultivierten Tierzelle nach Schritt (b), und danach Analysieren des mRNA-Levels des exprimierten FGF18 überwacht wird; und solche Testsubstanzen, welche die Funktion haben, die Expression von FGF18 zu inhibieren, in Schritt (d) ausgewählt werden durch Aussuchen von Systemen, welche niedrigere Level von FGF18-mRNA zeigten, als die, in welchen die Testsubstanz wirken gelassen wurde.

**Revendications**

1. Procédé permettant de promouvoir la pousse ou la repousse non-thérapeutique des cheveux, comportant le fait d'administrer une substance qui inhibe l'activité du facteur de croissance de fibroblastes FGF18, laquelle substance inhibant l'activité du FGF18 est :

   a) un peptide partiel de FGF18 ;
   b) un anticorps anti-FGF18 ;
   c) un vecteur d'expression dans lequel a été intégré un ADNc codant un peptide partiel de FGF18 doté d'une activité d'inhibition vis-à-vis de l'activité de FGF18 ;
   d) ou un vecteur d'expression dans lequel a été intégré un pARNi (petit ARN interférent) doté d'une activité d'inhibition vis-à-vis de l'expression de FGF18.

2. Procédé conforme à la revendication 1, dans lequel ladite substance inhibant l'activité du FGF18 est utilisée en combinaison avec un autre agent favorisant la pousse des cheveux ou un autre agent favorisant la repousse des cheveux.

3. Utilisation d'une substance qui inhibe l'activité du facteur de croissance de fibroblastes FGF18, laquelle substance inhibant l'activité du FGF18 est :

   a) un peptide partiel de FGF18 ;
   b) un anticorps anti-FGF18 ;
   c) un vecteur d'expression dans lequel a été intégré un ADNc codant un peptide partiel de FGF18 doté d'une activité d'inhibition vis-à-vis de l'activité de FGF18 ;
   d) ou un vecteur d'expression dans lequel a été intégré un pARNi (petit ARN interférent) doté d'une activité d'inhibition vis-à-vis de l'expression de FGF18 ;

   en vue de promouvoir la pousse ou la repousse non-thérapeutique des cheveux.

4. Utilisation conforme à la revendication 3, dans laquelle ladite substance inhibant l'activité du FGF18 est utilisée en combinaison avec un autre agent favorisant la pousse des cheveux ou un autre agent favorisant la repousse des cheveux.

5. Substance qui inhibe l'activité du facteur de croissance de fibroblastes FGF18, laquelle substance inhibant l'activité du FGF18 est :

   a) un peptide partiel de FGF18 ;
   b) un anticorps anti-FGF18 ;
   c) un vecteur d'expression dans lequel a été intégré un ADNc codant un peptide partiel de FGF18 doté d'une activité d'inhibition vis-à-vis de l'activité de FGF18 ;
   d) ou un vecteur d'expression dans lequel a été intégré un pARNi (petit ARN interférent) doté d'une activité d'inhibition vis-à-vis de l'expression de FGF18 ;

   pour utilisation dans un procédé de traitement d'une alopécie.

6. Substance pour utilisation conforme à la revendication 5, ladite substance inhibant l'activité du FGF18 étant utilisée en combinaison avec un autre agent favorisant la pousse des cheveux ou un autre agent favorisant la repousse des cheveux.

7. Substance pour utilisation conforme à la revendication 5, ladite substance étant utilisée en combinaison avec un autre agent thérapeutique pour alopécie.

8. Agent favorisant la pousse des cheveux, agent favorisant la repousse des cheveux, ou agent thérapeutique pour alopécie, comprenant en qualité d'ingrédient(s) actif(s) une substance qui inhibe l'activité du facteur de croissance de fibroblastes FGF18, laquelle substance inhibant l'activité du FGF18 est :

a) un peptide partiel de FGF18 ;
b) un anticorps anti-FGF18 ;
c) un vecteur d'expression dans lequel a été intégré un ADNc codant un peptide partiel de FGF18 doté d'une activité d'inhibition vis-à-vis de l'activité de FGF18 ;
d) ou un vecteur d'expression dans lequel a été intégré un pARNi (petit ARN interférent) doté d'une activité d'inhibition vis-à-vis de l'expression de FGF18.

**9.** Agent favorisant la pousse des cheveux, agent favorisant la repousse des cheveux, ou agent thérapeutique pour alopécie, conforme à la revendication 8, comprenant en outre un autre agent favorisant la pousse des cheveux, un autre agent favorisant la repousse des cheveux, ou un autre agent thérapeutique pour alopécie, respectivement.

**10.** Procédé *in vitro* de recherche par criblage d'une substance qui inhibe l'activité du facteur de croissance de fibroblastes FGF18, définie dans l'une des revendications 1 à 8, dans le but d'obtenir ainsi des candidats au rôle d'agent favorisant la pousse des cheveux, d'agent favorisant la repousse des cheveux ou d'agent thérapeutique pour alopécie, lequel procédé comporte les étapes suivantes (a) à (c) :

a) forcer l'expression d'au moins un gène de récepteur de FGF, choisi parmi les gènes *FGFR1c, FGFR2c, FGFR3c* et *FGFR4,* à la surface d'une cellule, par des techniques de génie génétique, et cultiver cette cellule ;
b) mettre une substance testée, conjointement avec du facteur FGF18, en contact avec le système cellulaire obtenu à l'issue de l'étape (a), où le récepteur de FGF est présent à la surface des cellules ;
c) et sélectionner parmi les substances testées celles qui font preuve, dans l'étape (b), d'une activité d'inhibition vis-à-vis de l'activité de promotion de la croissance cellulaire du facteur FGF18.

**11.** Procédé conforme à la revendication 10, dans lequel le récepteur de FGF est un récepteur FGFR3c ou FGFR4.

**12.** Procédé conforme à la revendication 10 ou 11, dans lequel la cellule à la surface de laquelle un récepteur de FGF est exprimé de manière forcée, selon la revendication 10, est une cellule de la souche de cellules IL-3-dépendantes Ba/F3 de souris.

**13.** Procédé de recherche par criblage d'une substance qui inhibe l'expression du facteur de croissance de fibroblastes FGF18, définie dans l'une des revendications 1 à 8, dans le but d'obtenir ainsi des candidats au rôle d'agent favorisant la pousse des cheveux, d'agent favorisant la repousse des cheveux ou d'agent thérapeutique pour alopécie, lequel procédé comporte les étapes suivantes (a) à (d) :

a) préparer une culture de cellules animales ou un animal d'expérience non-humain, capable d'exprimer le gène *FGF18* à un degré observable ;
b) mettre une substance testée en contact avec la culture de cellules animales issue de l'étape (a), ou mettre une substance testée en contact avec l'animal d'expérience non-humain issu de l'étape (a) ou administrer celle-là à celui-ci ;
c) surveiller l'expression du gène *FGF18,* après l'étape (b), dans la culture de cellules animales ou chez l'animal d'expérience non-humain ;
d) et sélectionner parmi les substances testées celles qui font preuve d'une fonction d'inhibition de l'expression du gène *FGF18.*

**14.** Procédé conforme à la revendication 13, dans lequel, dans l'étape (c), on surveille l'expression du gène *FGF18* en extrayant, après l'étape (b), de l'ARNm de la culture de cellules animales ou de l'animal d'expérience non-humain et en analysant ensuite le niveau de l'ARNm correspondant au gène *FGF18* exprimé, et dans lequel, dans l'étape (d), on sélectionne parmi les substances testées celles qui font preuve d'une fonction d'inhibition de l'expression du gène *FGF18* en choisissant les systèmes où les niveaux de l'ARNm correspondant au gène *FGF18* sont plus faibles que lorsqu'on ne fait pas agir la substance testée.

Fig. 1

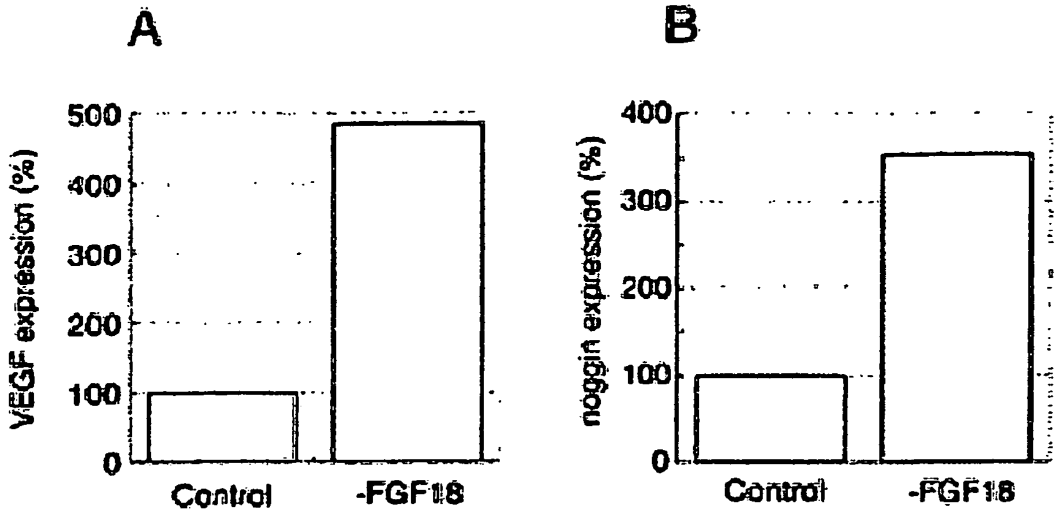
A
B
VEGF expression (%)
500
400
300
200
100
0
Control
-FGF18
noggin expression (%)
400
300
200
100
0
Control
-FGF18

Fig. 2

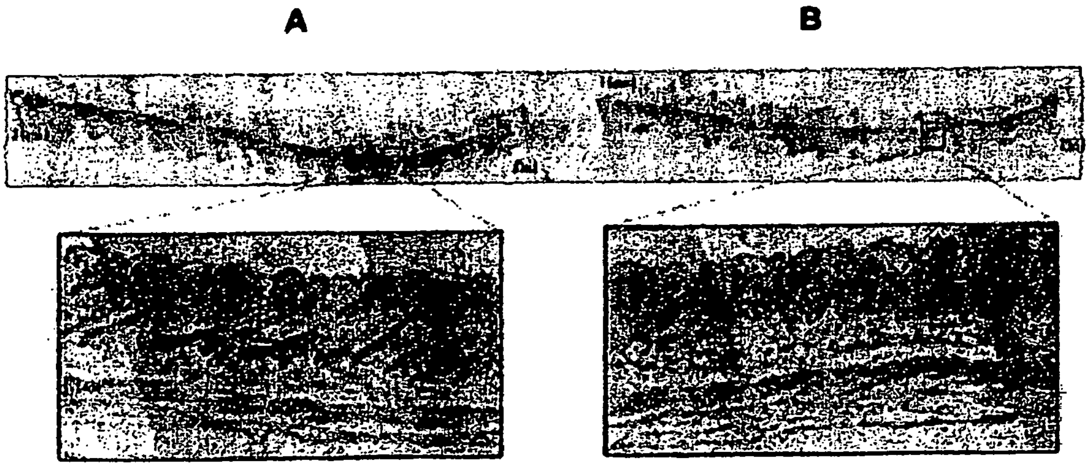
A
B

Fig. 3

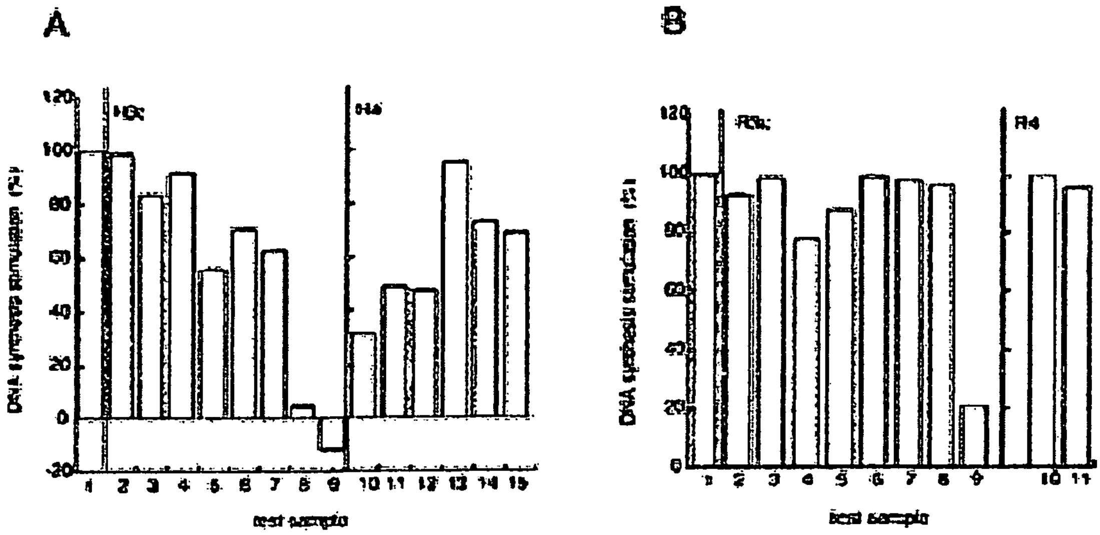
A
B
test sample

Fig. 4

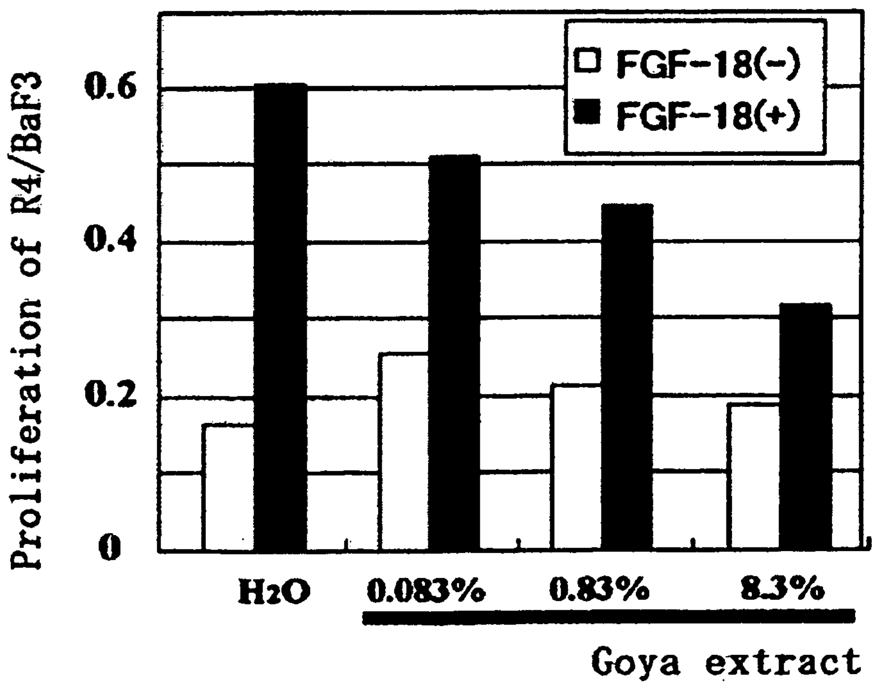
FGF-18(-)
FGF-18(+)
Proliferation of R4/BaF3
0.6
0.4
0.2
0
H2O
0.083%
0.83%
8.3%
Goya extract

Fig. 5

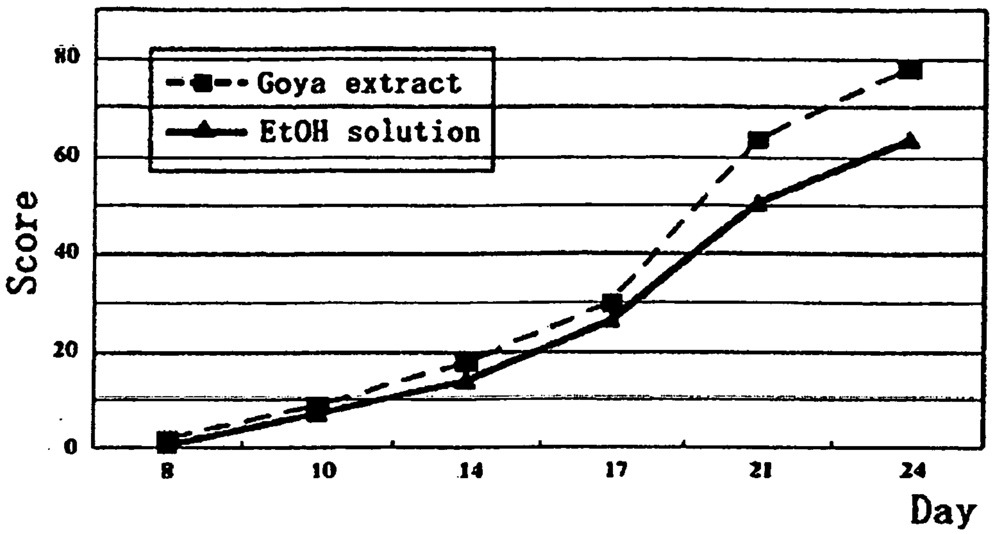
Goya extract
EtOH solution
Score
80
60
40
20
0
8
10
14
17
21
24
Day

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP HEI4224522 B
- JP 2006083082 A
- EP 08720756 A
- JP 2007038223 A

### Non-patent literature cited in the description


- **ORNITZ DM ; ITOH N.** *Fibroblast growth factors. Genome Biol2: REVIE WS3005,* 2001
- **DU CROS D.** Fibroblast growth factor and epidermal growth factor in hair development. *J Invest Dermatol,* 1993, vol. 101, 106S-113S
- **DU CROS DL ; ISAACS K ; MOORE GP.** Distribution of acidic and basicfibr oblast growth factorsin ovine skin during follicle morphogenesis. *J Cell Sci,* 1993, vol. 105, 667-674
- **HERBERT JM ; ROSENQUIST T ; GOTZ J ; MARTIN GR.** FGF5 as a regulator of the hair growth cycle: Evidence from targeted and spontaneous mutations. *Cell,* 1994, vol. 78, 1017-1025
- **DANILENKO DM ; RING BD ; YANAGIHARA D ; BENSON W ; WIEMANN B ; STARNESCO ; PIERCE GF.** Keratinocyte growth factor is an important endogenous mediator of hair follicle growth, development, and differentiation. *American J Pathol,* 1995, vol. 147, 145-154
- **MARCHESE C ; CHEDID M ; DIRSCH OR et al.** Modulation of keratinocyte growth factor and its receptor in reepithelializing human skin. *J Exp Med,* 1995, vol. 182, 1369-1376
- **GUO L ; DEGENSTEIN L ; FUCHS E.** keratinocyte growth factor is require dfor hair development but not for wound healing. *Genes Dev,* 1996, vol. 10, 165-175
- **ROSENQUIST TA ; MARTIN GR.** Fibroblast growth factor signalling in the hair growth cycle: Expression of the fibroblast growth factor receptor and ligand genes in the murine hair follicle. *Developmental Dynamics,* 1996, 379-386
- **PETHO-SCHRAMM A ; MULLER HJ ; PAUS R.** FGF5 and the murine hair cycle. *Arch Dermatol Res,* 1996, vol. 288, 264-266
- **MITSUI S ; OHUCHI A ; HOTTA M ; TSUBOI R ; OGAWA H.** Genes for a range of growth factors and cyclin-dependent kinase inhibitors are expressed by isolated human hair follicles. *Br J Dermatol,* 1997, vol. 137, 693-698
- **ORTEGA S ; ITTMANN M ; TSANG SH ; EHRLICH M ; BASILICO C.** Neuronaldefects and delayed wound healing in mice lacking fibroblast growth factor-2. *Proc Natl Acad Sci USA,* 1998, vol. 95, 5672-5677
- **SUZUKI S ; KATO T ; TAKIMOTO H et al.** Localization of rat FGF-5 protein in skin macrophage-like cells and FGF-5S protein in hair follicle: Possible involvement of two Fgf-5 gene products in hair growth cycleregulation. *J Invest Dermatol,* 1998, vol. 111, 963-972
- **SUZUKI S ; OTA Y ; OZAWA K ; IMAMURA T.** Dual-mode regulation of hair growth cycle by two Fgf-5 gene products. *J Invest Dermatol,* 2000, vol. 114, 456-463
- **NAKATAKE Y ; HOSHIKAWA M ; ASAKI T ; KASSAI Y ; ITOH N.** Identification of a novel fibroblast growth factor, FGF-22, preferentially expressed in the inner root sheath of the hair follicle. *Biochem Biophys Acta,* 2001, vol. 1517, 460-463
- **STENN KS ; PAUS R.** Controls of hair follicle cycling. *Physiol Rev,* 2001, vol. 81, 449-494
- **BEYER TA ; WERNER S ; DICKSON C ; GROSE R.** Fibroblast growth factor 22 and its potential role during skin development and repair. *Exp Cell Res,* 2003, vol. 287, 228-236
- **KAWANO M ; SUZUKI S ; SUZUKI M ; OKI J ; IMAMURA T.** Bulge- and basallayer-specific expression of fibroblast growth factor 13(FHF-2) in mouse skin. *J Invest Dermatol,* 2004, vol. 122, 1084-1090
- **KAWANO M ; KOMI-KURAMOCHI A ; ASADA M ; SUZUKI M ; OKI J ; JIANG J ; IMAMURA T.** Comprehensive analysis of FGF and FGFR expression in skin: FGF18 is highly expressed in hair follicles and capable of inducing anagen from telogenstage hair follicles. *J Invest Dermatol.,* 2005, vol. 124 (5), 877-885
- **ZHANGX ; IBRAHIMI OA ; OLSEN SK ; UMEMORI H ; MOHAMMADI M ; ORNITZ DM.** Receptor Specificity of the Fibroblast Growth Factor Family: THE COMPLETE MAMMALIAN FGFFAMILY. *J Biol Chem,* 2006, vol. 281 (23), 15694-15700

- *Nikkei Science,* April 1994, 20-45
- *Experimental Medicine Extra,* 1994, vol. 12 (15
- Basic Techniques for Gene Therapy. Experimental Medicine Separate. Yodo-sha, 1996
- **ORNITZ, DM. ; XU, J. ; COLVIN, JS. ; MCEWEN, DG ; MACARTHUR, CA. ; COULIER, F. ; GAO, G ; GOLDFARB, M.** Receptor specificity of the fibroblast growth factor family. *J. Biol. Chem.,* 1996, vol. 271 (25), 15292-15297
- **YONEDA, A. ; ASADA, M. ; ODA, Y ; SUZUKI, M. ; IMAMURA, T.** Engineering of an FGF-proteoglycan fusion protein with heparin-independent, mitogenic activity. *Nat. Biotec.,* 2000, vol. 18 (6), 641-644